# EUROPEAN PATENT SPECIFICATION

(11) **EP 4 259 124 B1**
(45) Date of publication and mention of the grant of the patent: **18.02.2026**
(21) Application number: 21835282.1
(22) Date of filing: 10.12.2021
(51) Int. Cl.: A61K 31/385, A61K 9/00, A61P 9/10

(54) **ANETHOLE TRITHIONE ADMINISTRATION REGIMEN FOR THE PREVENTION OR TREATMENT OF ISCHEMIA-REPERFUSION INJURIES**
ANETHOL-TRITHION-VERABREICHUNGSSCHEMA ZUR VORBEUGUNG ODER BEHANDLUNG VON ISCHÄMIE-REPERFUSIONSVERLETZUNGEN
RÉGIME D'ADMINISTRATION D'ANÉTHOLE TRITHIONE POUR LA PRÉVENTION OU LE TRAITEMENT DES LÉSIONS D'ISCHÉMIE-REPERFUSION

(30) Priority: 10.12.2020 US 202063123627 P; 24.02.2021 EP 21159033
(43) Date of publication of application: 18.10.2023
(73) Proprietor: Marin, Frédéric, 75011 Paris (FR)
(72) Inventor: LE GRAND, Bruno, 33604 Pessac Cedex (FR)
(74) Representative: Icosa
(86) International application number: PCT/EP2021/085258
(87) International publication number: WO 2022/123038

(56) References cited:
- WO-A1-2017/042267
- WO-A1-2020/049166
- BOUCARD A ET AL: "2818 Poster Session 5: Acute coronary syndrome treatment A specific complex I-induced ROS modulator, OP2113, is a new cardioproctective agent against acute myocardial infarction injuries during reperfusion", EUROPEAN HEART JOURNAL, 2 September 2019 (2019-09-02), pages 1 - 1, XP055824045, Retrieved from the Internet <URL:https://watermark.silverchair.com/ehz745.0990.pdf?token=AQECAHi208BE49Ooan9kkhW_Ercy7Dm3ZL_9Cf3qfKAc485ysgAAAwswggMHBgkqhkiG9w0BBwagggL4MIIC9AIBADCCAu0GCSqGSIb3DQEHATAeBglghkgBZQMEAS4wEQQMwvfBZdMNv4BOt2sdAgEQgIICvsF2s7BgLMzzbNRzKjv_0I62F_dM8pKVdluoPUn6nPO9ekP5GsX7JbixFHM1PxPIVf8ZbfOsclgT-ie1rKKzou15> [retrieved on 20210713]
- WANGDE DAI ET AL: "Abstract 13780: Effects of Op2113 on Myocardial Infarct Size and No Reflow in a Rat Myocardial Ischemia/reperfusion Model", CIRCULATION, VOL. 142, SUPPL 3, 12 November 2020 (2020-11-12), pages 1 - 5, XP055824076, Retrieved from the Internet <URL:https://www.ahajournals.org/doi/10.1161/circ.142.suppl_3.13780> [retrieved on 20210714]
- DAI WANGDE ET AL: "Effects of OP2113 on Myocardial Infarct Size and No Reflow in a Rat Myocardial Ischemia/Reperfusion Model", 8 February 2021 (2021-02-08), US, pages 1 - 11, XP055824044, ISSN: 0920-3206, Retrieved from the Internet <URL:https://link.springer.com/content/pdf/10.1007/s10557-020-07113-7.pdf> DOI: 10.1007/s10557-020-07113-7

## Description

### FIELD OF INVENTION

The present invention relates to the field of ischemia-reperfusion injuries. In particular, it relates to anethole trithione (AOL) for use in a method of preventing, treating, or lessening the severity or progression of, an ischemia-reperfusion injury in a human subject in need thereof, wherein:
a) a bolus of AOL is to be administered to the subject, said bolus comprising from 1.8 µg to 3 µg of AOL per kilogram oh the subject's weight, and
b) an intravenous (IV) infusion of AOL is to be administered to the subject immediately after the bolus of AOL, said IV infusion comprising from 2 µg to 6 µg of AOL per kg of the subject's weight per hour, to be administered continuously for a period of time of 6 hours or more,
wherein the concentration of AOL in the subject's plasma at steady-state during infusion is above 1 ng of AOL per milliliter (mL) of subject's plasma.

### BACKGROUND OF INVENTION

Despite advances in reperfusion therapy for acute ST elevation myocardial infarction (STEMI), mortality in some areas of the United States is as high as 8 % (Han *et al.,* **2019.** *J Am Heart Assoc.* **8**(**22**):e012054). In addition, heart failure remains a significant complication of myocardial infarction (Bahit *et al.,* **2018.** *JACC Heart Fail.* **6**(**3**):179-186). A potential therapy that might further improve outcomes following myocardial infarction, is to better protect the energy-producing factories of the cell: the mitochondria.

Several treatments have already been developed, among which anti-oxidant-based treatments, targeting reactive oxygen species (ROS) which production is triggered by reperfusion through the induction of oxidative stress. However, these treatments suffer several drawbacks: first, they only trap ROS after their production, hence after that these ROS have already caused their damages. Second, these anti-oxidants are non-specific, *i.e.,* they trap ROS regardless of their site of production in the cell. These ROS, in particular cytoplasmic ROS, are however crucial to cellular functioning, and are naturally present at low and stationary levels in normal cells.

Anethole trithione (AOL) was synthesized and used as a drug since 1696, and has been marketed in many countries and used in human therapy (Detaille et al., 2019. PLoS One. 14(5):e0216385). AOL, also named 5-(4-methoxyphenyl)-3H-1,2-dithiole-3-thione, does not act as an unspecific antioxidant molecule (*i.e.,* as a radical scavenger), but is a specific suppressor of mitochondrial superoxide/H₂O₂ production from complex I of the mitochondrial respiratory chain (Detaille *et al.,* **2019.** *PLoS One.* **14**(**5**):e0216385; Dias Amoedo *et al.,* **2020.** *Antioxid Redox Signal.* **33**(**13**):883-902). It has thus the capability to specifically inhibit the production of mitochondrial ROS (mtROS), without deregulating the fine-tuned levels of cytosolic ROS.

Here, the Inventors have estimated an optimal plasma concentration of AOL required to achieve a protective effect against ischemia-reperfusion injuries. AOL is however shortly metabolized into its principal metabolite, desmethylanethole trithione (AOX). Hence, the Inventors had to design an original sequence of administration to maintain this optimal plasma concentration, for preventing, treating, or lessening the severity or progression of, an ischemia-reperfusion injury.

WO2020/049166 A1 relates to a composition comprising anethole trithione or a derivative thereof, sulfobutyl ether-beta-cyclodextrin, and optionally a co-solvent, intended for use as a drug to treat or prevent free oxygen radical-related diseases.

Boucard A et al., 2019 relates to a large animal model of ischemia-reperfusion, anethole trithione (OP2113) reduced ST segment elevation, reduced troponin release, improved left ejection fraction and reduced infarct size.

Wangde Dai et al., 2020 describes that anethole trithione (OP2113) is a promising agent to reduce no-reflow as well as to reduce MI size.

### SUMMARY

The present invention relates to anethole trithione (AOL), for use in a method of preventing, treating, or lessening the severity or progression of, an ischemia-reperfusion injury in a human subject in need thereof, wherein:
a) a bolus of AOL is to be administered to the human subject, said bolus comprising from 1.8 µg to 3 µg of AOL per kilogram (kg) of the human subject's weight, and
b) an intravenous (IV) infusion of AOL is to be administered to the human subject immediately after the bolus of AOL, said IV infusion comprising from 2 µg to 6 µg of AOL per kg of the human subject's weight per hour, to be administered continuously for a period of time of 6 hours or more,
wherein the concentration of AOL in the subject's plasma at steady-state during infusion is above 1 ng of AOL per milliliter (mL) of subject's plasma, preferably the concentration of AOL in the subject's plasma at steady-state is assessed by liquid chromatography-mass spectrometry (LC/MS).

In one embodiment:
the bolus of AOL comprises at least 2 µg of AOL per kg of the human subject's weight, and the infusion comprises at least 3 µg of AOL per kg of the human subject's weight per hour to be administered continuously for a period of time of 6 hours or more; or
the bolus of AOL comprises at least 3 µg of AOL per kg of the human subject's weight, and the infusion comprises at least 2 µg of AOL per kg of the human subject's weight per hour to be administered continuously for a period of time of 6 hours or more; or
the bolus of AOL comprises at least 1.8 µg of AOL per kg of the human subject's weight, and the infusion comprises 6 µg of AOL per kg of the human subject's weight per hour to be administered continuously for a first period of time of 3 hours, then at least 2 µg, preferably at least 3 µg of AOL per kg of the human subject's weight per hour to be administered continuously for a second period of time of 3 hours or more.

In one embodiment, the concentration of AOL in the subject's plasma at steady-state during infusion ranges from 1 ng to 3 ng of AOL per mL of subject's plasma, preferably from 1 ng to 2.25 ng of AOL per mL of subject's plasma, preferably the concentration of AOL in the subject's plasma at steady-state is assessed by liquid chromatography-mass spectrometry (LC/MS).

In one embodiment, the bolus of AOL is to be administered prior to or at the time of reperfusion.

In one embodiment, the bolus of AOL is to be administered intravenously or intra-arterially, preferably by intracoronary injection.

In one embodiment, the infusion of AOL is to be administered continuously for 6 hours to 24 hours.

In one embodiment, the ischemia reperfusion injury occurs after ischemia and reperfusion of a tissue selected from the group comprising cardiac muscle tissue, skin tissue, skeletal muscle tissue, smooth muscle tissue, cartilage tissue, tendon tissue, brain tissue, spinal cord tissue, retinal tissue, corneal tissue, lung tissue, liver tissue, kidney tissue, pancreatic tissue, ovary tissue, testis tissue, intestinal tissue, stomach tissue, bladder tissue, or distal limb tissue.

In one embodiment, the ischemia reperfusion injury occurs after ischemia and reperfusion of the heart and wherein the ischemia-reperfusion injury is an acute coronary syndrome.

In one embodiment, the acute coronary syndrome is selected from the group comprising or consisting of ST elevation myocardial infarction (STEMI), non-ST elevation myocardial infarction (NSTEMI) and unstable angina. In one embodiment, the acute coronary syndrome is STEMI.

In one embodiment, preventing, treating, or lessening the severity or progression of the acute coronary syndrome comprises one or more of reducing reperfusion-induced ST-segment elevation, reducing troponin Ic release, reducing creatine phosphokinase (CPK) release, reducing infarct size, and increasing left ventricular ejection fraction.

In one embodiment, the human subject is undergoing surgery at the time of ischemia. In one embodiment, surgery is selected from the group comprising artery clamping and angioplasty.

In one embodiment, the human subject is undergoing tissue or organ transplantation at the time of ischemia. In one embodiment, tissue or organ transplantation is coronary artery bypass graft surgery.

### DEFINITIONS

**"About",** when preceding a figure, means plus or less 10 % of said figure value.

**"Anethole trithione"** or **"AOL"** or **"5-(4-methoxyphenyl)dithiole-3-thione"** refer to a substituted dithiolthione with the following formula:

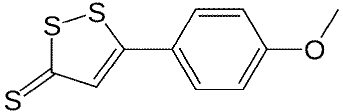

**"Bolus"** refers to the administration of a discrete amount of a medication, drug, or other compound (such as, *e.g.,* AOL) within a specific time, typically from about 30 seconds to about 30 minutes, in order to raise its concentration in blood to an effective level. The administration can be given by injection (such as, *e.g.*, intravenously, intra-arterially [*e.g*., intracoronarily], intramuscularly, intrathecally or subcutaneously; preferably intravenously or intra-arterially intracoronarily [*e.g*., intracoronarily]; more preferably intravenously), or by inhalation.

**"Infusion"** refers to the continuous intravenous administration of a medication, drug, or other compound (such as, *e.g.,* AOL) over a long period of time, typically from about 30 minutes to several hours or days, in order to maintain its concentration in blood to an effective level, *i.e.,* a **"steady-state"** level.

**"Ischemia-reperfusion injury",** also termed **"reoxygenation injury"** or **"reperfusion injury",** refers to damages caused to organs or tissues when blood supply returns to said organ or tissue (*i.e.,* during reperfusion) after a period of restriction in blood supply (*i.e.,* after ischemia). Indeed, the absence of oxygen and nutrients from blood during ischemia creates a condition in which the reperfusion triggers further damages through neutrophil infiltration, interference with cellular ion homeostasis, mitochondrial dysfunction and oxidative damage through the induction of oxidative stress rather than, or along with, restoration of normal function. Any tissue can be subject to ischemia-reperfusion injury, as long as this tissue has been subjected to both a phase of ischemia and a phase of reperfusion. Ischemia-reperfusion injury can occur after a spontaneously occurring event, such as, *e.g.,* an arterial blockage, or after a planned event, such as, *e.g.,* a surgical intervention (during arterial clamping, angioplasty, transplantation surgery, etc.) or therapeutic treatment (during thrombolytic therapy, etc.).

**"Lessen the severity or progression", "lessening of the severity or progression"** and any declinations thereof, with reference to ischemia-reperfusion injuries, refer to the partial alleviation, inhibition, or mitigation of the ischemia-reperfusion injury and/or of symptoms associated therewith.

**"Prevent", "prevention"** and any declinations thereof, with reference to ischemia-reperfusion injuries, refer to the reduction or elimination of the occurrence of the ischemia-reperfusion injury and/or of symptoms associated therewith.

**"Steady-state"** generally refers to the property of a system that tends to maintain a stable and constant condition over time. Also referred to as **"homeostasis",** it refers in the present invention to a state where the concentration of anethole trithione (AOL) in the subject's plasma reaches a stable and constant plateau during the intravenous (IV) infusion of AOL to said subject.

**"Symptoms associated with an ischemia-reperfusion injury"** refer to symptoms observed in ischemic tissues, or in tissues damaged by ischemia; or more globally, to symptoms experienced by a subject suffering from an ischemia-reperfusion injury. Examples of such symptoms include, without limitation, a decreased blood flow, a hypoxia, an anoxia, a hypoglycemia, a decreased metabolism, an increased necrosis, and/or an increased apoptosis of the ischemic tissue as compared to non-ischemic tissue. Further symptoms associated with an ischemia-reperfusion injury include, without limitation, cramping, claudication, numbness, tingling, weakness, pain, reduced wound healing, inflammation, skin discoloration, and/or gangrene.

**"Treat", "treatment",** and any declinations thereof, with reference to ischemia-reperfusion injuries, refer to the complete alleviation, inhibition, onset delay, or curation of the ischemia-reperfusion injury and/or of symptoms associated therewith.

### DETAILED DESCRIPTION

The references to the methods of treatment by therapy of this description are to be interpreted as references to compound of the present invention for use in those methods.

This invention also relates to anethole trithione (AOL), for use in a method of preventing, treating, or lessening the severity or progression of, an ischemia-reperfusion injury in a human subject in need thereof, wherein:
a) a bolus of AOL is to be administered to the subject, said bolus comprising from 1.8 µg to 3 µg of AOL per kilogram (kg) of the subject's weight, and
b) an intravenous (IV) infusion of AOL is to be administered to the subject immediately after the bolus of AOL, said IV infusion comprising from 2 µg to 6 µg of AOL per kg of the subject's weight per hour, to be administered continuously for a period of time of 6 hours or more,
wherein the concentration of AOL in the subject's plasma at steady-state during infusion is above 1 ng of AOL per milliliter (mL) of subject's plasma, preferably the concentration of AOL in the subject's plasma at steady-state is assessed by liquid chromatography-mass spectrometry (LC/MS).

Another aspect of the disclosure relates to anethole trithione (AOL), for use in a method of preventing, treating, or lessening the severity or progression of, an ischemia-reperfusion injury in a subject in need thereof, preferably in a human subject in need thereof, comprising the separate, sequential or concomitant administration to the subject of:
a) a bolus of AOL, and
b) an intravenous (IV) infusion of AOL,
wherein the concentration of AOL in the subject's plasma at steady-state during infusion is above about 1 ng of AOL per milliliter (mL) of subject's plasma.

According to the invention, a bolus of anethole trithione (AOL) is administered or is to be administered to the subject, as defined in the claims.

In one embodiment, the bolus of AOL is administered or is to be administered prior to reperfusion, *i.e.,* before blood supply returns to the ischemic tissue.

In one embodiment, the bolus of AOL is administered or is to be administered about 48 hours, 24 hours, 18 hours, 12 hours, 6 hours, 5 hours, 4 hours, 3 hours, 2 hours, 1 hour, 55 minutes, 50 minutes, 45 minutes, 40 minutes, 35 minutes, 30 minutes, 25 minutes, 20 minutes, 15 minutes, 10 minutes, or 5 minutes prior to reperfusion, *i.e.,* 48 hours, 24 hours, 18 hours, 12 hours, 6 hours, 5 hours, 4 hours, 3 hours, 2 hours, 1 hour, 55 minutes, 50 minutes, 45 minutes, 40 minutes, 35 minutes, 30 minutes, 25 minutes, 20 minutes, 15 minutes, 10 minutes, or 5 minutes before blood supply returns to the ischemic tissue.

In one embodiment, the bolus of AOL is administered or is to be administered about 4 hours prior to reperfusion, *i.e.,* 4 hours before blood supply returns to the ischemic tissue.

In one embodiment, the bolus of AOL is administered or is to be administered about 3 hours prior to reperfusion, *i.e.,* 3 hours before blood supply returns to the ischemic tissue.

In one embodiment, the bolus of AOL is administered or is to be administered about 2 hours prior to reperfusion, *i.e.,* 2 hours before blood supply returns to the ischemic tissue.

In one aspect of the disclosure, the bolus of AOL comprises from about 0.3 µg to about 4.0 µg of AOL per kilogram (kg) of the subject's weight. In one embodiment, the bolus of AOL comprises from about 0.9 µg to about 4.0 µg of AOL per g) of the subject's weight. In one embodiment, the bolus of AOL comprises from about 1.8 µg to about 4.0 µg of AOL per kg of the subject's weight. In one embodiment, the bolus of AOL comprises from about 2.0 µg to about 4.0 µg of AOL per kg of the subject's weight.

In one aspect of the disclosure, the bolus of AOL comprises from about 0.3 µg to about 3.0 µg of AOL per kg of the subject's weight. In one embodiment, the bolus of AOL comprises from about 0.9 µg to about 3.0 µg of AOL per kg of the subject's weight. In one embodiment, the bolus of AOL comprises from 1.8 µg to 3.0 µg of AOL per kg of the subject's weight. In one embodiment, the bolus of AOL comprises from 2.0 µg to 3.0 µg of AOL per kg of the subject's weight.

According to the invention, the bolus of AOL comprises from 1.8 µg to 3.0 µg of AOL per kg of the subject's weight.

In one embodiment, the bolus of AOL comprises from 2.0 µg to 3.0 µg of AOL per kg of the subject's weight.

In one aspect of the disclosure, the bolus of AOL comprises about 0.3 µg, 0.4 µg, 0.5 µg, 0.6 µg, 0.7 µg, 0.8 µg, 0.9 µg, 1.0 µg, 1.1 µg, 1.2 µg, 1.3 µg, 1.4 µg, 1.5 µg, 1.6 µg, 1.7 µg, 1.8 µg, 1.9 µg, 2.0 µg, 2.1 µg, 2.2 µg, 2.3 µg, 2.4 µg, 2.5 µg, 2.6 µg, 2.7 µg, 2.8 µg, 2.9 µg, 3.0 µg, 3.1 µg, 3.2 µg, 3.3 µg, 3.4 µg, 3.5 µg, 3.6 µg, 3.7 µg, 3.8 µg, 3.9 µg, or 4.0 µg of AOL per kg of the subject's weight.

In one aspect of the disclosure, the bolus of AOL comprises at least 1.8 µg of AOL per kg of the subject's weight. In one embodiment, the bolus of AOL comprises at least about 2 µg of AOL per kg of the subject's weight. In one aspect of the disclosure, the bolus of AOL comprises at least about 3 µg of AOL per kg of the subject's weight.

In one embodiment, the bolus of AOL comprises 1.8 µg of AOL per kg of the subject's weight. In one embodiment, the bolus of AOL comprises 2 µg of AOL per kg of the subject's weight. In one embodiment, the bolus of AOL comprises about 3 µg of AOL per kg of the subject's weight.

In one embodiment, the bolus of AOL is administered or is to be administered by intravenous (IV) or by intraarterial (IA) route.

In one embodiment, the bolus of AOL is administered or is to be administered by intravenous (IV) route.

In one embodiment, the bolus of AOL is administered or is to be administered by intraarterial (IA) route.

Examples of IA routes include, but are not limited to:
- administration in the ascending aorta, including, but not limited to, administration in the coronary ostium (including right and left coronary ostia), in the left coronary artery (including the left anterior descending artery and the circumflex artery) or in the right coronary artery;
- administration in the aortic arch, including, but not limited to, administration in the brachiocephalic artery, in the left or right common carotid arteries (including branches thereof such as the internal carotid artery and the external carotid artery), or in the left or right subclavian arteries (including branches thereof such as the vertebral arteries, the internal thoracic artery, the thyrocervical trunk, the costocervical trunk, the dorsal scapular artery and the axillary arteries);
- administration in the descending thoracic aorta, including, but not limited to, administration in the bronchial arteries;
- administration in the abdominal aorta, including, but not limited to, administration in the celiac artery (including branches thereof such as the left gastric artery, the common hepatic artery and the splenic artery), in the superior mesenteric artery, in the renal arteries, or in the inferior mesenteric artery;
- administration in the common iliac arteries, including branches thereof such as the external or internal iliac arteries, the femoral arteries, the profunda femoris arteries, the popliteal arteries, the peroneal arteries, the anterior or posterior tibial arteries, and the dorsalis pedis arteries.

A preferred example of IA routes is administration in the left or right coronary arteries, including their ostia, or in branches thereof.

According to the invention, an intravenous (IV) infusion of anethole trithione (AOL) is administered or is to be administered to the subject.

In one embodiment, the IV infusion of AOL is administered or is to be administered from the time of reperfusion.

In one aspect of the disclosure, the IV infusion of AOL is administered or is to be administered after the bolus of AOL, such as, *e.g.,* in a separate, sequential, or concomitant manner, preferably in a separate or sequential manner.

According to the invention, the IV infusion of AOL is administered or is to be administered directly or immediately after the bolus of AOL, such as, *e.g.,* less than 1 minute, 2 minutes, 3 minutes, 4 minutes, 5 minutes, 10 minutes, 15 minutes, 20 minutes, 25 minutes, 30 minutes, 45 minutes, or 60 minutes after the end of the bolus administration.

In one aspect of the disclosure, the IV infusion of AOL comprises from about 0.4 µg to about 6.0 µg of AOL per kg of the subject's weight per hour. In one embodiment, the IV infusion of AOL comprises from about 1.7 µg to about 6.0 µg of AOL per kg of the subject's weight per hour.

According to the invention, the IV infusion of AOL comprises from 2.0 µg to 6.0 µg of AOL per kg of the subject's weight per hour.

In one aspect of the disclosure, the IV infusion of AOL comprises from about 0.4 µg to about 4.0 µg of AOL per kg of the subject's weight per hour. In one aspect of the disclosure, the IV infusion of AOL comprises from about 1.7 µg to about 4.0 µg of AOL per kg of the subject's weight per hour. In one embodiment, the IV infusion of AOL comprises from 2.0 µg to 4.0 µg of AOL per kg of the subject's weight per hour.

In one embodiment, the IV infusion of AOL comprises from 2.0 µg to less than 4.0 µg of AOL per kg of the subject's weight per hour.

In one aspect of the disclosure, the IV infusion of AOL comprises about 0.4 µg, 0.5 µg, 0.6 µg, 0.7 µg, 0.8 µg, 0.9 µg, 1.0 µg, 1.1 µg, 1.2 µg, 1.3 µg, 1.4 µg, 1.5 µg, 1.6 µg, 1.7 µg, 1.8 µg, 1.9 µg, 2.0 µg, 2.1 µg, 2.2 µg, 2.3 µg, 2.4 µg, 2.5 µg, 2.6 µg, 2.7 µg, 2.8 µg, 2.9 µg, 3.0 µg, 3.1 µg, 3.2 µg, 3.3 µg, 3.4 µg, 3.5 µg, 3.6 µg, 3.7 µg, 3.8 µg, 3.9 µg, 4.0 µg, 4.1 µg, 4.2 µg, 4.3 µg, 4.4 µg, 4.5 µg, 4.6 µg, 4.7 µg, 4.8 µg, 4.9 µg, 5.0 µg, 5.1 µg, 5.2 µg, 5.3 µg, 5.4 µg, 5.5 µg, 5.6 µg, 5.7 µg, 5.8 µg, 5.9 µg, or 6.0 µg of AOL per kg of the subject's weight per hour.

In one embodiment, the IV infusion of AOL comprises at least 2 µg of AOL per kg of the subject's weight per hour. In one embodiment, the IV infusion of AOL comprises at least 3 µg of AOL per kg of the subject's weight per hour. In one embodiment, the IV infusion of AOL comprises at least 6 µg of AOL per kg of the subject's weight per hour.

In one embodiment, the IV infusion of AOL comprises 2 µg of AOL per kg of the subject's weight per hour. In one embodiment, the IV infusion of AOL comprises 3 µg of AOL per kg of the subject's weight per hour. In one embodiment, the IV infusion of AOL comprises 6 µg of AOL per kg of the subject's weight per hour.

According to the invention, the IV infusion of AOL is administered or is to be administered continuously for at least about 6 hours. In one aspect of the disclosure, the IV infusion of AOL is administered or is to be administered continuously for about 3 hours to about 24 hours, such as, for about 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, or 24 hours; preferably for about 6 hours to about 24 hours.

In one embodiment, the IV infusion of AOL is administered or is to be administered continuously for 6 hours. In one embodiment, the IV infusion of AOL is administered or is to be administered continuously for 12 hours. In one embodiment, the IV infusion of AOL is administered or is to be administered continuously for 24 hours.

In one embodiment, the IV infusion of AOL is administered or is to be administered at a higher concentration for a first period of time, then at a lower concentration for a second period of time, it being understood that AOL is administered or is to be administered over this first and second period of time, together, for at least about 6 hours as described above.

In one embodiment:
- a higher concentration of AOL comprises at least 3 µg of AOL per kg of the subject's weight per hour; in particular, a higher concentration of AOL comprises from 3.0 µg to 6.0 µg of AOL per kg of the subject's weight per hour, such as 3.0 µg, 3.1 µg, 3.2 µg, 3.3 µg, 3.4 µg, 3.5 µg, 3.6 µg, 3.7 µg, 3.8 µg, 3.9 µg, 4.0 µg, 4.1 µg, 4.2 µg, 4.3 µg, 4.4 µg, 4.5 µg, 4.6 µg, 4.7 µg, 4.8 µg, 4.9 µg, 5.0 µg, 5.1 µg, 5.2 µg, 5.3 µg, 5.4 µg, 5.5 µg, 5.6 µg, 5.7 µg, 5.8 µg, 5.9 µg, or 6.0 µg of AOL per kg of the subject's weight per hour; preferably a higher concentration of AOL comprises 6.0 µg of AOL per kg of the subject's weight per hour; and
- a lower concentration of AOL comprises at least 2 µg of AOL per kg of the subject's weight per hour; in particular, a lower concentration of AOL comprises from 2.0 µg to 4.0 µg of AOL per kg of the subject's weight per hour, such as 2.0 µg, 2.1 µg, 2.2 µg, 2.3 µg, 2.4 µg, 2.5 µg, 2.6 µg, 2.7 µg, 2.8 µg, 2.9 µg, 3.0 µg, 3.1 µg, 3.2 µg, 3.3 µg, 3.4 µg, 3.5 µg, 3.6 µg, 3.7 µg, 3.8 µg, 3.9 µg, or 4.0 µg of AOL per kg of the subject's weight per hour; preferably a lower concentration of AOL comprises from 2.0 µg to 3.0 µg of AOL per kg of the subject's weight per hour; more preferably a lower concentration of AOL comprises 3.0 µg of AOL per kg of the subject's weight per hour.

In one embodiment, the first period of time and second period of time each independently from each other last at least 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours or 23 hours, it being understood that the sum of the first period of time and second period of time is at least 6 hours.

In one embodiment, the first period of time lasts for 3 hours. In one embodiment, the second period of time lasts for 3 hours or more.

In one embodiment, the IV infusion of AOL during the second period of time is administered or is to be administered directly or immediately after the IV infusion of AOL during the first period of time, such as, *e.g.,* less than 1 minute, 2 minutes, 3 minutes, 4 minutes, 5 minutes, 10 minutes, 15 minutes, 20 minutes, 25 minutes, 30 minutes, 45 minutes, or 60 minutes after the end of the first period of time. Preferably, the IV infusion of AOL is administered or is to be administered continuously over the first and second period of time, *i.e.,* without interruption between the first and second period of time.

In one aspect of the disclosure:
- the bolus of AOL comprises from about 0.3 µg to about 4.0 µg of AOL per kilogram (kg) of the subject's weight. In one embodiment, the bolus of AOL comprises from about 0.9 µg to about 4.0 µg of AOL per g) of the subject's weight. In one embodiment, the bolus of AOL comprises from about 1.8 µg to about 4.0 µg of AOL per kg of the subject's weight. In one embodiment, the bolus of AOL comprises from about 2.0 µg to about 4.0 µg of AOL per kg of the subject's weight; and
- the IV infusion of AOL comprises from about 0.4 µg to about 6.0 µg of AOL per kg of the subject's weight per hour. In one embodiment, the IV infusion of AOL comprises from about 1.7 µg to about 6.0 µg of AOL per kg of the subject's weight per hour. In one embodiment, the IV infusion of AOL comprises from about 2.0 µg to about 6.0 µg of AOL per kg of the subject's weight per hour.

In one aspect of the disclosure:
- the bolus of AOL comprises from about 0.3 µg to about 3.0 µg of AOL per kg of the subject's weight. In one embodiment, the bolus of AOL comprises from about 0.9 µg to about 3.0 µg of AOL per kg of the subject's weight. In one embodiment, the bolus of AOL comprises from about 1.8 µg to about 3.0 µg of AOL per kg of the subject's weight. In one embodiment, the bolus of AOL comprises from about 2.0 µg to about 3.0 µg of AOL per kg of the subject's weight; and
- the IV infusion of AOL comprises from about 0.4 µg to about 4.0 µg of AOL per kg of the subject's weight per hour. In one embodiment, the IV infusion of AOL comprises from about 1.7 µg to about 4.0 µg of AOL per kg of the subject's weight per hour. In one embodiment, the IV infusion of AOL comprises from about 2.0 µg to about 4.0 µg of AOL per kg of the subject's weight per hour.

In one embodiment:
- the bolus of AOL comprises from 2.0 µg to 3.0 µg of AOL per kg of the subject's weight; and
- the IV infusion of AOL comprises from 2.0 µg to less than 4.0 µg of AOL per kg of the subject's weight per hour.

In one aspect of the disclosure:
- the bolus of AOL comprises about 0.3 µg, 0.4 µg, 0.5 µg, 0.6 µg, 0.7 µg, 0.8 µg, 0.9 µg, 1.0 µg, 1.1 µg, 1.2 µg, 1.3 µg, 1.4 µg, 1.5 µg, 1.6 µg, 1.7 µg, 1.8 µg, 1.9 µg, 2.0 µg, 2.1 µg, 2.2 µg, 2.3 µg, 2.4 µg, 2.5 µg, 2.6 µg, 2.7 µg, 2.8 µg, 2.9 µg, 3.0 µg, 3.1 µg, 3.2 µg, 3.3 µg, 3.4 µg, 3.5 µg, 3.6 µg, 3.7 µg, 3.8 µg, 3.9 µg, or 4.0 µg of AOL per kg of the subject's weight; and
- the IV infusion of AOL comprises about 0.4 µg, 0.5 µg, 0.6 µg, 0.7 µg, 0.8 µg, 0.9 µg, 1.0 µg, 1.1 µg, 1.2 µg, 1.3 µg, 1.4 µg, 1.5 µg, 1.6 µg, 1.7 µg, 1.8 µg, 1.9 µg, 2.0 µg, 2.1 µg, 2.2 µg, 2.3 µg, 2.4 µg, 2.5 µg, 2.6 µg, 2.7 µg, 2.8 µg, 2.9 µg, 3.0 µg, 3.1 µg, 3.2 µg, 3.3 µg, 3.4 µg, 3.5 µg, 3.6 µg, 3.7 µg, 3.8 µg, 3.9 µg, 4.0 µg, 4.1 µg, 4.2 µg, 4.3 µg, 4.4 µg, 4.5 µg, 4.6 µg, 4.7 µg, 4.8 µg, 4.9 µg, 5.0 µg, 5.1 µg, 5.2 µg, 5.3 µg, 5.4 µg, 5.5 µg, 5.6 µg, 5.7 µg, 5.8 µg, 5.9 µg, or 6.0 µg of AOL per kg of the subject's weight per hour.

According to the invention, the concentration of AOL in the subject's plasma at steady-state during infusion, *i.e.,* after administration of the bolus of AOL and during the IV infusion of AOL, is above 1 ng of AOL per milliliter (mL) of subject's plasma.

In one embodiment, the concentration of AOL in the subject's plasma at steady-state during infusion ranges from 1 ng to 3 ng of AOL per mL of subject's plasma.

In one embodiment, the concentration of AOL in the subject's plasma at steady-state during infusion ranges from 1 ng to 2.25 ng of AOL per mL of subject's plasma.

In one embodiment, the concentration of AOL in the subject's plasma at steady-state during infusion ranges from 1 ng to 2 ng of AOL per mL of subject's plasma.

In one embodiment, the concentration of AOL in the subject's plasma at steady-state during infusion is of 1.0 ng, 1.1 ng, 1.2 ng, 1.3 ng, 1.4 ng, 1.5 ng, 1.6 ng, 1.7 ng, 1.8 ng, 1.9 ng, 2.0 ng, 2.1 ng, 2.2 ng, 2.3 ng, 2.4 ng, 2.5 ng, 2.6 ng, 2.7 ng, 2.8 ng, 2.9 ng, or 3.0 ng of AOL per mL of subject's plasma.

The concentration of AOL in the subject's plasma can be measured by means and methods well known to the skilled artisan, such as liquid chromatography-mass spectrometry (LC/MS) or any other suitable means or method. For example, a blood sample from the subject can be centrifuged - preferably within 30 minutes after blood sampling - at about 1 500 g for 10 minutes at 2-8°C to obtain plasma - the skilled artisan is familiar with method of obtaining plasma from a blood sample, and can readily adapt the centrifugal force, time, temperature and any other parameter according to his routine practice. Immediately after centrifugation, the plasma sample can be analyzed to assess the concentration of AOL by liquid chromatography-mass spectrometry (LC/MS).

In one embodiment, AOL is formulated for administration by injection (such as, *e.g.,* intravenously, intra-arterially [*e.g.,* intracoronarily], intramuscularly, intrathecally or subcutaneously; preferably intravenously or intra-arterially intracoronarily [*e.g*., intracoronarily]; more preferably intravenously), or by inhalation.

In one embodiment, AOL is formulated in a composition comprising sulfobutyl ether-beta-cyclodextrin. In one embodiment, AOL is formulated in a composition comprising sulfobutyl ether-beta-cyclodextrin with a molar ratio of sulfobutyl ether-beta-cyclodextrin:AOL ranging from about 10 to about 400, preferably from about 57 to about 200. In one embodiment, the amount of the sulfobutyl ether-beta-cyclodextrin ranges from about 1 to about 40 % (w/w), preferably from about 2.5 to about 30 % (w/w), more preferably from about 5 to about 20 % (w/w) in weight relative to the total weight of the composition. In one embodiment, the composition further comprises at least one buffer selected from the group comprising or consisting of citrate buffer, acetate buffer, tris(hydroxymethyl)aminomethane and phosphate buffer, preferably the buffer is a phosphate buffer. In one embodiment, the composition has a pH ranging from about 6 to about 7.8, preferably from about 6.5 to about 7.5.

In one embodiment, the ischemia-reperfusion injury occurs after ischemia and reperfusion of a tissue selected from the group comprising or consisting of cardiac muscle tissue (a.k.a. heart tissue), skin tissue, skeletal muscle tissue, smooth muscle tissue, cartilage tissue, tendon tissue, brain tissue or spinal cord tissue (a.k.a. central nervous system tissue and parts thereof), retinal tissue, corneal tissue, lung tissue, liver tissue, kidney tissue, pancreatic tissue, ovary tissue, testis tissue, intestinal tissue (a.k.a. bowel tissue), stomach tissue, bladder tissue, or distal limb tissue (including arms and legs).

In other words, the ischemic tissue, or the tissue damaged by ischemia, is selected from the group comprising or consisting of cardiac muscle tissue (a.k.a. heart tissue), skin tissue, skeletal muscle tissue, smooth muscle tissue, cartilage tissue, tendon tissue, brain tissue or spinal cord tissue (a.k.a. central nervous system tissue and parts thereof), retinal tissue, corneal tissue, lung tissue, liver tissue, kidney tissue, pancreatic tissue, ovary tissue, testis tissue, intestinal tissue (a.k.a. bowel tissue), stomach tissue, bladder tissue, or distal limb tissue (including arms and legs).

In one embodiment, the ischemia-reperfusion injury is selected from the group comprising or consisting of acute coronary syndrome, acute lung injury (ALI), acute myocardial infarction (AMI), acute respiratory distress syndrome (ARDS), arterial occlusive disease, arteriosclerosis, articular cartilage defect, aseptic systemic inflammation, atherosclerotic cardiovascular disease, autoimmune disease, bone fracture, bone fracture, brain edema, brain hypoperfusion, Buerger's disease, burns, cancer, cardiovascular disease, cartilage damage, cerebral infarct, cerebral ischemia, cerebral stroke, cerebrovascular disease, chemotherapy-induced neuropathy, chronic infection, chronic mesenteric ischemia, claudication, congestive heart failure, connective tissue damage, contusion, coronary artery disease (CAD), critical limb ischemia (CLI), Crohn's disease, deep vein thrombosis, deep wound, delayed ulcer healing, delayed wound-healing, type I diabetes, type II diabetes, diabetic neuropathy, diabetes-induced ischemia, disseminated intravascular coagulation (DIC), embolic brain ischemia, frostbite, graft-versus-host disease (GVHD), hereditary hemorrhagic telangiectasia, ischemic vascular disease, hyperoxic injury, hypoxia, inflammation, inflammatory bowel disease, inflammatory disease, injured tendons, intermittent claudication, intestinal ischemia, ischemia, ischemic brain disease, ischemic heart disease, ischemic peripheral vascular disease, ischemic placenta, ischemic renal disease, ischemic vascular disease, ischemic-reperfusion injury, laceration, left main coronary artery disease, limb ischemia, lower extremity ischemia, myocardial infarction, myocardial ischemia, organ ischemia, osteoarthritis, osteoporosis, osteosarcoma, Parkinson's disease, peripheral arterial disease (PAD), peripheral artery disease, peripheral ischemia, peripheral neuropathy, peripheral vascular disease, pre-cancer, pulmonary edema, pulmonary embolism, remodeling disorder, renal ischemia, retinal ischemia, retinopathy, sepsis, skin ulcers, solid organ transplantation, spinal cord injury, stroke, subchondral-bone cyst, thrombosis, thrombotic brain ischemia, tissue ischemia, transient ischemic attack (TIA), traumatic brain injury, ulcerative colitis, vascular disease of the kidney, vascular inflammatory conditions, von Hippel-Lindau syndrome, or wounds to tissues or organs.

In one embodiment, the ischemic tissue, or the tissue damaged by ischemia, has any one or several of the following symptoms: decreased blood flow, hypoxia, anoxia, hypoglycemia, decreased metabolism, increased necrosis, and increased apoptosis compared to non-ischemic tissue.

In one embodiment, symptoms associated with the ischemic tissue, or with the tissue damaged by ischemia, are selected from the group comprising or consisting of cramping, claudication, numbness, tingling, weakness, pain, reduced wound healing, inflammation, skin discoloration, and gangrene.

In one embodiment, where the ischemia-reperfusion injury occurs after ischemia and reperfusion of the cardiac muscle tissue (a.k.a. heart tissue), the ischemia-reperfusion injury is an acute coronary syndrome. In some embodiments, complications arising from acute coronary syndrome include, without limitation, arrhythmia, atrial fibrillation, ventricular fibrillation, ventricular tachycardia, atrial flutter, conduction disturbances, papillary muscle dysfunction and/or mural thrombosis.

In one embodiment, the acute coronary syndrome is selected from the group comprising or consisting of ST elevation myocardial infarction (STEMI), non-ST elevation myocardial infarction (NSTEMI) and unstable angina.

In one embodiment, where the ischemia-reperfusion injury occurs after ischemia and reperfusion of the brain tissue (a.k.a. central nervous system and parts thereof), the ischemia-reperfusion injury is stroke.

In one embodiment, where the ischemia-reperfusion injury occurs after ischemia and reperfusion of the lung tissue, the ischemia-reperfusion injury is pulmonary edema and/or pulmonary hypertension.

In one embodiment, where the ischemia-reperfusion injury occurs after ischemia and reperfusion of the liver tissue, the ischemia-reperfusion injury is liver failure.

In one embodiment, where the ischemia-reperfusion injury occurs after ischemia and reperfusion of the kidney tissue, the ischemia-reperfusion injury is kidney failure.

In one embodiment, the ischemia-reperfusion is chronic.

In one embodiment, the ischemia-reperfusion is acute.

In one embodiment, the subject has had or is undergoing at the time of ischemia, surgery, chemotherapy, radiation therapy, coronary artery bypass graft, or a cell, tissue, or organ transplantation.

In particular, ischemia-reperfusion is an inevitable event accompanying some surgery requiring artery clamping.

The method of the disclosure is therefore well suitable for preventing, treating, or lessening the severity or progression of, an ischemia-reperfusion injury during such artery clamping procedure.

Ischemia-reperfusion is also an inevitable event accompanying some surgery requiring an angioplasty. Angioplasty (a.k.a. balloon angioplasty or percutaneous transluminal angioplasty) is an endovascular procedure during which a deflated balloon attached to a catheter is passed over a guide-wire into the narrowed vessel and then inflated to a fixed size. The balloon forces expansion of the blood artery and the surrounding muscular wall, allowing an improved blood flow. The balloon is then deflated and withdrawn. Ischemia therefore occurs when the balloon is inflated, temporarily stopping the blood flow to the organ or tissue normally irrigated by this artery.

The method of the disclosure is therefore well suitable for preventing, treating, or lessening the severity or progression of, an ischemia-reperfusion injury during such angioplasty procedure.

Ischemia-reperfusion is also an inevitable event accompanying cell, tissue, or organ transplantation. One specific example of organ transplantation is coronary artery bypass graft surgery (also known in the art as coronary artery bypass surgery, CABG surgery, heart bypass or bypass surgery), during which a blood vessel from the patient, typically a vein from the leg, is removed and grafted to the coronary artery to bypass a blockage in the coronary artery.

The method of the disclosure is therefore well suitable for preventing, treating, or lessening the severity or progression of, an ischemia-reperfusion injury during cell, tissue, or organ transplantation. In particular, the method of the disclosure is well suitable for preventing, treating, or lessening the severity or progression of, an ischemia-reperfusion injury during coronary artery bypass graft surgery.

In particular, two distinct periods of ischemia occur during cell, tissue, or organ transplantation: and (1) ischemia during cell, tissue, or organ retrieval, *i.e.,* from the time of cross clamping (or of asystole in non-heart-beating donors), until cold perfusion is started; and (2) ischemia during implantation, *i.e.,* from removal of the cell, tissue, or organ from ice until reperfusion.

The method of the disclosure applies to both these periods of ischemia. For example, it is conceivable that the method of the disclosure be applied to the donor (*e.g*., with a bolus of AOL administered prior to cell, tissue, or organ retrieval and an IV infusion of AOL administered during tissue or organ retrieval); and/or to the recipient (*e.g*., with a bolus of AOL administered prior to clamping and an IV infusion of AOL administered during reperfusion of the grafted tissue or organ).

Some additional aspects of the disclosure are disclosed hereafter.

The present disclosure relates on anethole trithione (AOL), for use in a method of preventing, treating, or lessening the severity or progression of, an ischemia-reperfusion injury in a subject in need thereof, wherein:
a) a bolus of AOL is to be administered to the subject, and
b) an intravenous (IV) infusion of AOL is to be administered to the subject,
wherein the concentration of AOL in the subject's plasma at steady-state during infusion ranges from about 1 ng to about 2 ng of AOL per milliliter (mL) of subject's plasma.

The present disclosure relates on AOL for use according to the present disclosure, wherein the bolus of AOL is to be administered prior to or at the time of reperfusion.

The present disclosure relates on AOL for use according to the present disclosure, wherein the infusion of AOL is to be administered immediately after the bolus of AOL.

The present disclosure relates on AOL for use according to the present disclosure, wherein the bolus of AOL is to be administered intravenously or intra-arterially, preferably by intracoronary injection.

The present disclosure relates on AOL for use according to the present disclosure, wherein the bolus of AOL comprises from about 2 µg to about 3 µg of AOL per kilogram (kg) of the subject's weight, preferably the bolus of AOL comprises about 2 µg of AOL per kg of the subject's weight.

The present disclosure relates on AOL for use according to the present disclosure, wherein the infusion of AOL comprises from about 2 µg to less than about 4 µg of AOL per kg of the subject's weight per hour, preferably the infusion of AOL comprises about 3 µg of AOL per kg of the subject's weight per hour.

The present disclosure relates on AOL for use according to the present disclosure, wherein the infusion of AOL is to be administered continuously for about 3 hours to about 24 hours.

The present disclosure relates on AOL for use according to the present disclosure, wherein the ischemia-reperfusion injury occurs after ischemia and reperfusion of a tissue selected from the group comprising cardiac muscle tissue, skin tissue, skeletal muscle tissue, smooth muscle tissue, cartilage tissue, tendon tissue, brain tissue, spinal cord tissue, retinal tissue, corneal tissue, lung tissue, liver tissue, kidney tissue, pancreatic tissue, ovary tissue, testis tissue, intestinal tissue, stomach tissue, bladder tissue, or distal limb tissue.

The present disclosure relates on AOL for use according to the present disclosure, wherein the ischemia-reperfusion injury occurs after ischemia and reperfusion of the heart and wherein the ischemia-reperfusion injury is an acute coronary syndrome.

The present disclosure relates on AOL for use according to the present disclosure, wherein the acute coronary syndrome is selected from the group comprising or consisting of ST elevation myocardial infarction (STEMI), non-ST elevation myocardial infarction (NSTEMI) and unstable angina.

The present disclosure relates on AOL for use according to the present disclosure, wherein the acute coronary syndrome is STEMI.

The present disclosure relates on AOL for use according to the present disclosure, wherein preventing, treating, or lessening the severity or progression of the acute coronary syndrome comprises one or more of reducing reperfusion-induced ST-segment elevation, reducing troponin Ic release, reducing creatine phosphokinase (CPK) release, reducing infarct size, and increasing left ventricular ejection fraction.

The present disclosure relates on AOL for use according to the present disclosure, wherein the subject is undergoing surgery at the time of ischemia.

The present disclosure relates on AOL for use according to the present disclosure, wherein surgery is selected from the group comprising artery clamping and angioplasty.

The present disclosure relates on AOL for use according to the present disclosure, wherein the subject is undergoing tissue or organ transplantation at the time of ischemia.

### BRIEF DESCRIPTION OF THE DRAWINGS

**Figures 1A-B** are a set of graphs showing the infarct size and no-reflow size in the three groups (preventive, curative, and control). **Figure 1A****:** regression of the myocardial infarct mass *versus* the risk zone mass; **Figure 1B****:** regression of the no-reflow mass *versus* the risk zone mass.
**Figure 2** is a graph showing the effect of AOL plasma concentrations inferior (<) or superior (>) to 1 ng/mL at the time of reperfusion on reperfusion-induced ST-segment elevation.
**Figure 3** is a graph showing the effect of AOL plasma concentrations inferior (<) or superior (>) to 1 ng/mL at the time of reperfusion on troponin Ic plasma level.
**Figure 4** is a graph showing the effect of AOL plasma concentrations inferior (<) or superior (>) to 1 ng/mL at the time of reperfusion on creatine phosphokinase (CPK) plasma level.
**Figure 5** is a graph showing the effect of AOL plasma concentrations inferior (<) or superior (>) to 1 ng/mL at the time of reperfusion on infarct size.
**Figures 6A-B** are a set of two graphs showing the effect of AOL plasma concentrations inferior (<) or superior (>) to 1 ng/mL at the time of reperfusion on ejection fraction. **Figure 6A****:** left ventricular ejection fraction (LVEF) at day 3; **Figure 6B****:** normalized LVEF.
**Figure 7** is a graph showing the mean AOL plasma concentrations from a clinical pharmacokinetics study over first 12 hours following one of 4 dosage regimens comprising a bolus IV administration and a 1-step IV infusion.
**Figures 8A-B** are a set of two graphs showing the proportionality between AOL plasma concentration *versus* bolus or infusion doses. **Figure 8A****:** between AOL plasma concentration at the peak and bolus dose. **Figure 8B****:** between AOL plasma concentration at the end of infusion and infusion dose.

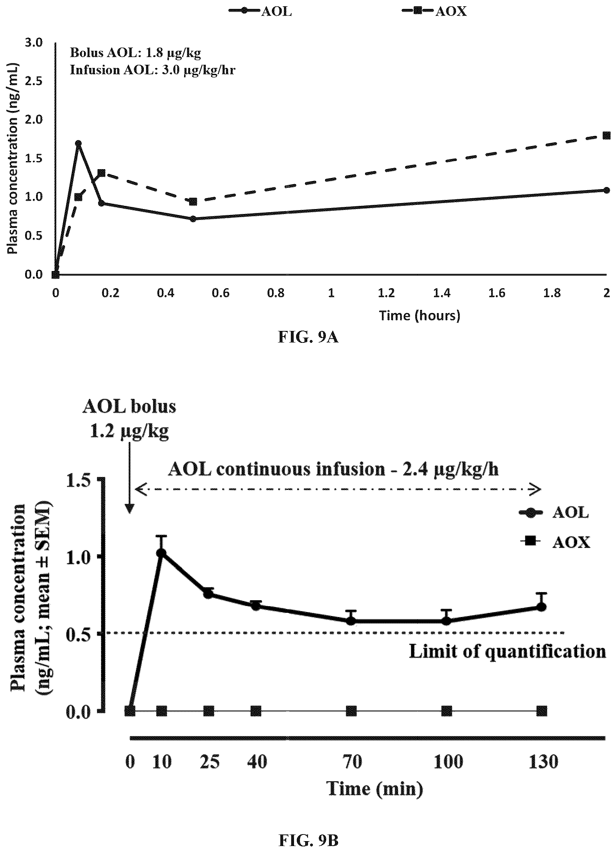

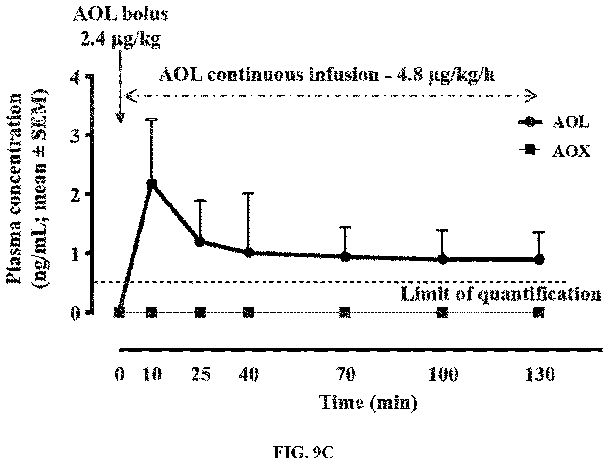

**Figure 9A-C** are a set of three graphs showing the plasma concentration of AOL and its main metabolite, AOX (in ng/mL) over the first couple of hours in human and sheep upon AOL bolus and infusion. **Figure 9A****:** AOL (solid line) and AOX (dotted line) plasma concentration in human, in the course of a 1.8 µg/kg bolus immediately followed by a 3.0 µg/kg/hour infusion. **Figure 9B****:** AOL (thick line with round marks) and AOX (thin line with square marks) plasma concentration in sheep, in the course of a 1.2 µg/kg bolus immediately followed by a 2.4 µg/kg/hour infusion. The dotted line represents the limit of quantification of AOL and AOX. **Figure 9C****:** AOL (thick line with round marks) and AOX (thin line with square marks) plasma concentration in sheep, in the course of a 2.4 µg/kg bolus immediately followed by a 4.8µg/kg/hour infusion. The dotted line represents the limit of quantification of AOL and AOX.
**Figure 10** is a graph showing AOL plasma concentration (in ng/mL) in human, during a 2-step infusion protocol immediately following a bolus. Three regimens are shown: in dotted line with triangle marks, a 1-step infusion protocol immediately following a bolus as a comparison point; and in solid line with round marks and dotted line with square marks, a 2-step infusion protocol immediately following a bolus at two different dosages.
**Figures 11A-B** are a set of two graphs showing the influence of infusion duration on the concentration of mitochondrial ROS (in arbitrary units [a.u.]). **Figure 11A****:** peak mitochondrial ROS concentration as a function of AOL infusion duration. **Figure 11B****:** mitochondrial ROS dynamic at the end of the infusion, for three infusion durations from 0 to 24/48 hours.
**Figures 12A-B** are a set of two graphs showing the maximal target threshold of AOL per mL of plasma in human. **Figure 12A****:** peak mitochondrial ROS concentration (in arbitrary units [a.u.]) as a function of AOL target plasma concentration (in ng/mL). **Figure 12B****:** left ventricular ejection fraction (in %) as a function of AOL target plasma concentration (in ng/mL).

### EXAMPLES

The present invention is further illustrated by the following examples.

### Example 1: effects of AOL in an in vivo ischemia-reperfusion model in rat

### Material and Methods

### Animals

64 female Sprague Dawley rats (~ 6 months old) were anesthetized with intraperitoneal ketamine (90 mg/kg) and xylazine (10 mg/kg). Only female rats were used in this study as it was previously reported that gender had no effect on the extent of myocardial injury in the setting of experimental myocardial infarction in rats (Li & Kloner, 1995. J Thromb Thrombolysis. 2(3):221-225); and that lethal reperfusion-induced arrhythmias led to a higher mortality in male rats *versus* female rats (Dow et al., 2015. Springerplus. 4:96).

The animals were intubated and mechanically ventilated with room air at respiratory rate of 60 cycles/minute and tidal volume of 1 mL per 100 g body weight (Harvard Apparatus Rodent Ventilator, Model 683, South Natick, Mass). A water circulating heating pad was used to keep the rat body temperature at around 37°C. Their neck surgical area was shaved and cleaned, and catheters were inserted into the jugular vein (for drug delivery) and carotid artery (for arterial waveform monitoring) via cut downs. Under clean conditions, the chest cavity was opened through an incision in the left 4^{th} intercostal space to expose the heart. The pericardium was gently removed to expose the anterior surface of the left ventricle. A 4-0 silk suture was placed under the proximal portion of the left coronary artery as it runs through the interventricular groove just under the tip of the left atrial appendage. The end of the suture was threaded through a small plastic tube to make a snare for coronary artery occlusion. At the time of occlusion, the artery was pulled through the plastic tube and the tube was clamped. Coronary artery reperfusion occurred by releasing the clamp and watching the surface of the heart for reactive hyperemia. The left coronary artery was occluded for 30 minutes followed by 3 hours of reperfusion.

### Randomization and AOL administration

Rats were randomized into one of the 3 groups:
- preventive group (n = 22): AOL infusion starting 5 minutes before coronary artery occlusion;
- curative group (n = 20): AOL infusion starting 5 minutes prior to reperfusion;
- control group (n = 22): vehicle infusion starting 5 minutes before coronary artery occlusion.

The investigators were blinded to the animal randomization. Infusions in all groups were continuous throughout the end of the study (h+3 after reperfusion). In treated rats, the dose of AOL was 0.2 mg/kg/hour delivered in saline at a rate of infusion of 2 mL/kg/hour.

### Hemodynamics and histological data

By the end of the 3-hour reperfusion, a solution of thioflavin S was injected into the jugular vein during the last one minute of reperfusion to assess the distribution of the no-reflow phenomenon (also known as "zone of microvascular obstruction"). Thioflavin S is a dye that appears fluorescent in areas receiving perfusion, when heart slices are visualized under UV light; while the no-reflow area, that lacks perfusion, appears dark (non-fluorescent). At the end of the period of reperfusion after thioflavin S injection, the proximal coronary artery was briefly re-occluded and blue dye (Super imperse blue) was injected into the jugular vein. Blue dye circulates only to perfused areas and does not reach the ischemic zone (which appears pink when viewing heart slices under standard white light).

At the end of this step, the animal was euthanized under deep anesthesia with ketamine/xylazine by intravascular injection of KCl (149 mg/mL) at 0.5 mL/250 g body weight in order to stop the heart in a relative diastolic or relaxed state. The heart was excised and was gently washed in clear saline, and then transected into 4 transverse slices from apex to base.

The heart slices were photographed under white light in order to delineate the ischemic risk zone (pink) *versus* the non-ischemic regions (blue) that received the blue dye. The heart slices were then photographed under UV light in order to delineate the areas of perfusion by thioflavin S (fluorescent areas) *versus* the no-reflow zones (non-fluorescent).

Finally, hearts were incubated in 1 % of triphenyl tetrazolium chloride (TTC), a chemical that stains viable cells brick red, while dead or necrotic cells appear white. The photographs were used for planimetry in order to determine the percentage of each heart slice that was at risk, infarcted, or contained no-reflow. Planimetered photos were corrected for the weight of each heart slice and then the percentage of the mass of each left ventricle that was at risk (ischemic), demonstrated no-reflow, and was necrotic, was calculated. Infarct size was expressed as the percentage of the ischemic risk zone that went on to develop ischemic necrosis. The no-reflow zone was also expressed as a percent of the left ventricular risk zone, but also as percentage of the zone of necrosis.

Rectal temperature, heart rate and blood pressure were monitored throughout the surgical procedure.

### Statistical analysis

All data are reported as mean ± SEM. Values between the groups were compared with one-way analysis of variance (ANOVA). Statistically significant differences were established at p < 0.05. If an ANOVA three-way comparison was significant, then two-way comparisons were performed using Holm-Sidak method.

### Results

Out of the 64 rats, three rats died at around 2 hours after coronary artery reperfusion (1 in the control group and 2 in the preventive group). Another 3 rats were excluded due to no ischemic risk area available, because their heart stopped beating at the time of blue dye injection (1 rat in each of the 3 groups).

Overall statistical analysis by ANOVA showed that there was a significant difference in the area of no-reflow/area at risk (%) and the area of necrosis/area at risk (%) among the 3 groups (**Table 1**).

**Table 1: ischemic risk area, infarct and no-reflow size in the control and 2 treated groups. #: significant difference between control versus curative (p = 0.043); *: significant difference between control versus preventive (p = 0.026). &: significant difference between control versus preventive (p < 0.05). LV: left ventricular.**

| **Groups** | **Control** | **Curative** | **Preventive** | ***p* value** |
|---|---|---|---|---|
| **Number of rats** | 20 | 19 | 19 | |
| **Area at risk/LV area (%)** | 50.9±1.6 | 54.4±1.5 | 48.8±2.1 | 0.072 |
| **Area of no-reflow/Area at risk (%)** | 38.9±3.1 | 30.1±2.3^{#} | 28.8±2.4* | 0.017 |
| **Area of necrosis/Area at risk (%)** | 57.0±3.6 | 50.8±3.9 | 44.5±2.9^{&} | 0.010 |

There was no statistically significant difference in the ischemic risk areas among the 3 groups.

The no-reflow area was completely overlap with area of infarction, not outside irreversibly injured tissue, and was significantly smaller in the 2 treated groups compared to the control group.

The infarct size was significantly reduced in the preventive group compared to the control group. There was also a trend toward smaller infarct size in the curative *versus* the control group.

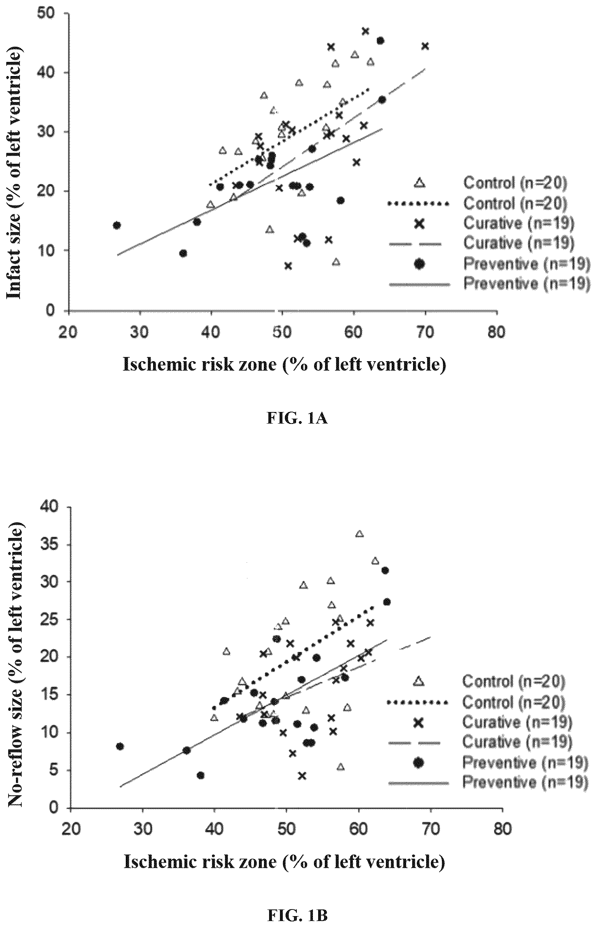

**Fig. 1A** plots the regression line of the myocardial infarct mass *versus* the risk zone mass and shows that for any size of the risk zone, the size of the infarct is reduced in the preventive group with a positive trend downward in the curative group compared to vehicle control. **Fig. 1B** plots the regression line of no-reflow mass *versus* risk zone mass and shows that AOL therapy (both preventive and curative) shifted the line downward, such that for any given risk zone mass, the size of the no-reflow mass was less than in the vehicle group.

Hemodynamics, including heart rate and arterial blood pressure, were reported at 3 time points: (1) prior to coronary artery occlusion (baseline); (2) prior to coronary artery reperfusion (at the end of 30 minutes of coronary artery occlusion); and (3) at the end of 3 hours of coronary artery reperfusion. There were no differences in heart rate and arterial blood pressure among the 3 groups during the procedure.

### Conclusion

In conclusion, when AOL was administered in a preventive fashion (*i.e.,* prior to coronary artery occlusion), it significantly reduced both the size of the no-reflow zone (by 26 %) as well as the size of the myocardial infarction (by 22 %).

When AOL was given in a curative fashion (*i.e.,* shortly before reperfusion), it significantly reduced the size of the no-reflow zone (by 23 %), and reduced the size of the myocardial infarction (by 11 %). The benefits of AOL were observed without effect on hemodynamics. This reduction remains substantial as even 5 % reductions in infarct size translate to improvements in heart function and clinical outcomes (Stone *et al.,* **2016.** *J Am Coll Cardiol.* **67**(**14**):1674-83).

### Example 2: effect of AOL in an in vivo ischemia-reperfusion model in sheep

### Materials and Methods

Here, we investigated the effect of AOL in an *in vivo* ischemia-reperfusion model in sheep, by administering AOL prior to reperfusion to assess the cardioprotective potential of AOL. Several parameters were evaluated:
- the infarct size, determined at the end of the experiment (day 3), and assessed by two independent pathologists;
- the plasmatic exposure, evaluated by dosing plasma samples collected at regular intervals after administration of AOL by mass spectrometry;
- ECG parameters; and
- heart contractility, assessed through the measurement of the ejection fraction.

The efficacy of AOL was evaluated by comparing these parameters in vehicle- and AOL-treated groups using the sheep model.

Sheep underwent a myocardial ischemia-reperfusion, while either receiving AOL or the vehicle as a control. The animals underwent a three-day follow-up period and were euthanized on day 3.

Myocardial ischemia followed by reperfusion was induced by using the balloon catheter procedure, which consists in occluding the distal left anterior descending (LAD) coronary artery by inflating/deflating a balloon. This particular procedure allowed to have infarction sizes highly reproducible and to occlude only temporally the coronary artery.

AOL or vehicle were administered as a bolus intravenous (IV) injection 10 minutes prior to reperfusion. This bolus injection was expected to ensure an effective AOL plasma exposure on reperfusion and was immediately followed by a continuous IV infusion starting at the beginning of the reperfusion, for a total duration of administration (bolus + infusion) of 130 minutes.

Different groups were included in the study, but the analysis was focused on the cardiac endpoints as a function of plasma concentrations of the active substance (AOL) achieved following IV AOL administration, regardless of the dose administered.

### Results

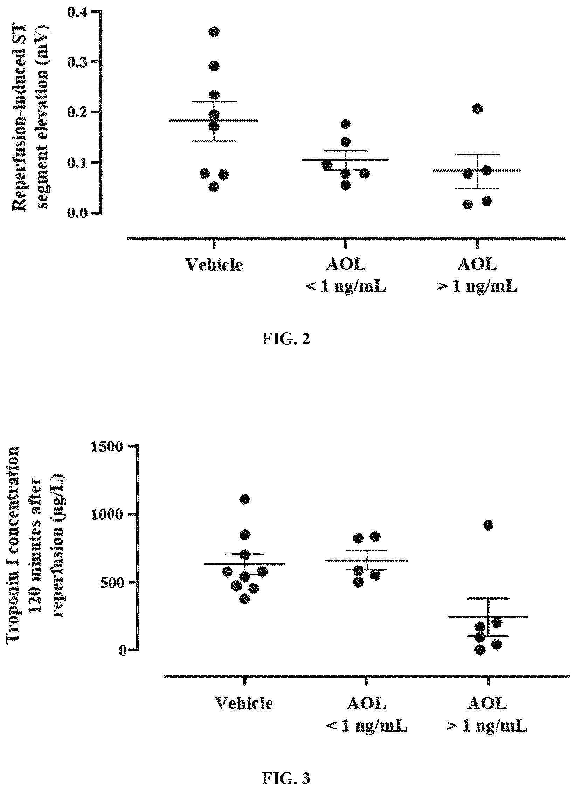

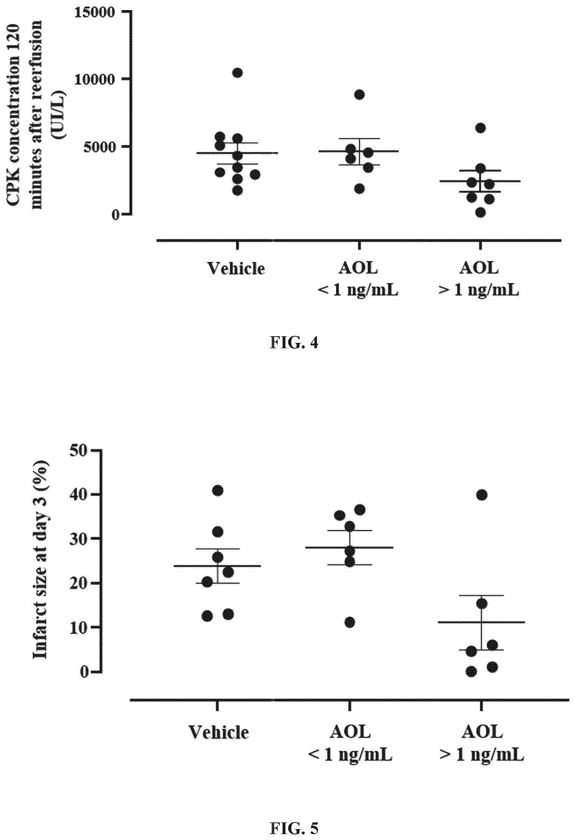

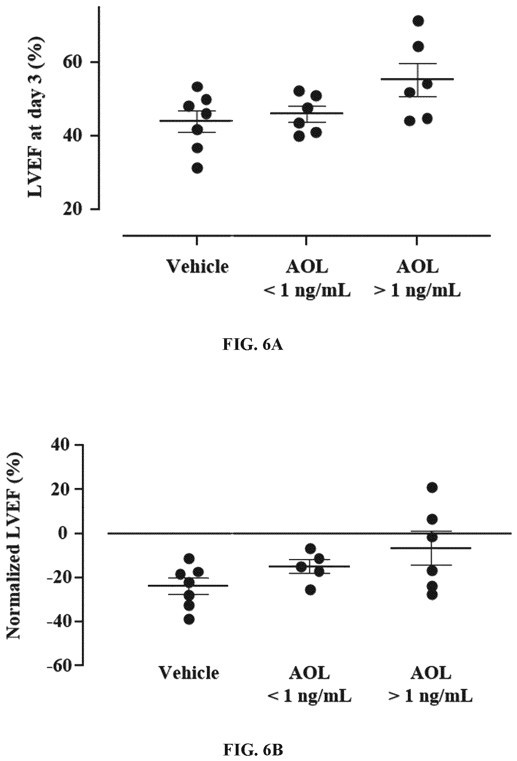

A clear relationship between plasma concentration of AOL and the positive impact on a range of cardiac parameters was observed in this preclinical proof-of-concept study in sheep. These cardiac parameters include reperfusion-induced ST-segment elevation (**Fig. 2**), troponin Ic release (**Fig. 3**), creatine phosphokinase (CPK) release (**Fig. 4**), infarct size (**Fig. 5**), and ejection fraction (**Figs. 6A** and **6B**).

However, this evaluation highlighted the importance of reaching an appropriate plasma AOL level at the time of reperfusion by administering a bolus, since the positive impact compared to vehicle was observed when levels of AOL were superior to 1 ng per mL of plasma when the occluding balloon was deflated, *i.e.,* at reperfusion. This deflation mimics percutaneous coronary intervention (PCI) in patients following acute ST segment elevation myocardial infarction (STEMI).

### Example 3: pharmacokinetics study

AOL plasma levels and timing of administration is essential to protect the myocardium from reperfusion injury.

The large-animal model efficacy testing conducted in the preclinical program (Example 2) demonstrated that cardiac function was improved particularly when plasma levels of AOL were superior to 1 ng/mL at the time of reperfusion of the myocardium.

Thus, in the targeted clinical application of the treatment of myocardial reperfusion injury, it is critical that plasma levels of AOL are at an adequate efficacious level at the time of percutaneous coronary intervention (PCI), and thus have reached this level by very early at the beginning of the reperfusion.

Based on preclinical data (Example 2), we estimated that plasma levels of AOL need to be between about 1 and 2 ng/mL.

### Materials and Methods

A single site, randomized, placebo-controlled, open-label and single blind, ascending dose, cross-over study primarily aiming at evaluating the pharmacokinetics, safety and tolerability of AOL administered intravenously in healthy volunteers *versus a* marketed formulation of AOL (Sulfarlem S25^{®}) administered orally was carried out at QPS Netherlands B.V. (Groningen, The Netherlands). Twelve healthy male and female subjects aged between 18 and 65 years old were included in this study.

During this clinical pharmacokinetics study, several dosage regimens have been tested to assess the safety of AOL and to better characterized its pharmacokinetics profile, when administered by IV route (**Table 2**).

**Table 2: overall summary of AOL plasma exposure following 4 different dosage regimens (bolus + 1-step infusion). Mean bolus volume and mean flow rate were estimated using a mean body weight of 70 kg.**

| **AOL administration** | | | | **AOL plasma concentration** | | |
|---|---|---|---|---|---|---|
| **Dosing** | **AOL concentration (µg/mL)** | **Mean bolus volume (mL)** | **Mean flow rate (mL/hr)** | **Cₘₐₓ (ng/mL)** | **Valley (ng/mL)** | **End of 12-hour infusion (IV) (ng/mL)** |
| **Bolus:** 0.3 µg/kg | 10 | 2.1 | 2.8 | 0.29 | ~0.08 | ~0.1 |
| **Infusion:** 0.4 µg/kg/hr for 12 hours | | | | | | |
| **Bolus:** 0.9 µg/kg | 100 | 0.6 | 1.2 | 1.04 | ~0.37 | ~0.8 |
| **Infusion:** 1.7 µg/kg/hr for 12 hours | | | | | | |
| **Bolus:** 1.8 µg/kg | 100 | 1.3 | 2.1 | 1.94 | ~0.72 | ~1.7 |
| **Infusion:** 3 µg/kg/hr for 12 hours | | | | | | |
| **Bolus:** 4 µg/kg | 100 | 2.8 | 4.2 | 4.14 | ~2.67 | ~3.5 |
| **Infusion:** 6 µg/kg/hr for 12 hours | | | | | | |

### Results

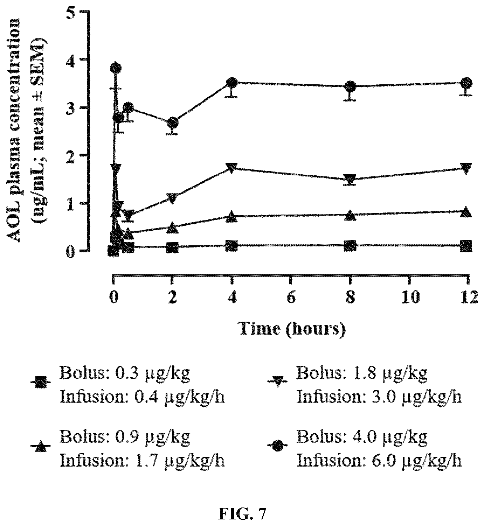

Resulting AOL plasma exposures are reported in **Table 2** and illustrated on **Fig. 7****.**

Data showed that the pharmacokinetics profile of AOL is characterized by:
- a peak, that appears immediately after administration of the bolus directly into the blood stream;
- a valley (or trough) between the peak and steady state, which is a combination of AOL delivered by the bolus injection being rapidly metabolized into its principal metabolite, desmethylanethole trithione (AOX), and the delivery of AOL from the infusion which takes time to reach steady state as it is also being metabolized;
- a plateau, corresponding to the achievement of steady state AOL concentration that is maintained until the end of the infusion, and represents an equilibrium between delivery of AOL and its degradation.

As seen in **Fig. 7****,** the steady state was reached by around 3 hours for all dosage regimens tested. This original sequence of a bolus immediately followed by a continuous IV infusion guaranteed that AOL plasma levels remained above the targeted 1 to 2 ng AOL per mL of plasma threshold, in spite of its rapid degradation.

### Example 4: AOL plasma concentration in human using modeling

The data of Example 3 showed that, in the clinical conditions, the plasma concentration of AOL at the peak is proportional to the bolus dose, and that the plasma concentration of AOL at the end of infusion is proportional to the infusion dose (correlation bolus/Cₘₐₓ: r² = 0.9987; p = 0.0007; correlation infusion/end of infusion: r² = 0.9990; p = 0.0005).

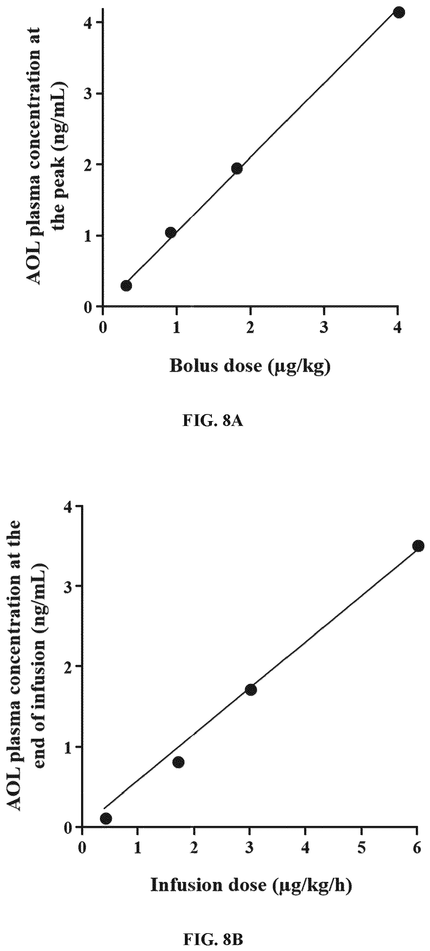

The equation of the linear regression showing the bolus dose proportionality of the plasma concentration at the Cₘₐₓ (Y) is **Y** = **1.046** × **X,** where X is the bolus dose (**Fig. 8A**). The equation of the linear regression showing the infusion dose proportionality of the plasma concentration at the end of infusion (Y) is **Y** = **0.5723** × **X,** where X is the infusion dose (**Fig. 8B**).

A pharmacokinetic two-compartment open model of AOL was built from the observed data of the 3 highest dosage regimens tested in Example 3. This model was used to predict the anticipated doses to be used in human. Results are given in **Table 3.**

From these modeling data, to maintain AOL plasma levels above the targeted 1 to 2 ng AOL per mL of plasma threshold, it appears that regimens that would be particularly suitable comprise:
- a bolus ranging from about 2 to about 3 µg of AOL per kilogram (kg) of the subject's weight, followed by
- a 12-hour infusion ranging from about 2 to less than 4 µg of AOL per kilogram (kg) of the subject's weight per hour (such as, *e.g.,* 3 µg/kg/h).

Preferably, the bolus should remain the lowest possible while meeting the targeted threshold of AOL plasma concentration.

**Table 3: predicted AOL plasma concentrations (in ng/mL) using modeling following an administration regimen comprising a 30-second bolus IV injection followed by a 12-hour continuous IV infusion.**

| **Total AOL dose (µg)** | 1921.7 | 2808.7 | 3695.6 | 5469.5 | 1995.6 | 2882.6 | 3769.5 | 5543.4 | |
|---|---|---|---|---|---|---|---|---|---|
| **Time from first administration (hours)** | **Bolus:** 2 µg/kg | **Bolus:** 2 µg/kg | **Bolus:** 2 µg/kg | **Bolus:** 2 µg/kg | **Bolus:** 3 µg/kg | **Bolus:** 3 µg/kg | **Bolus:** 3 µg/kg | **Bolus:** 3 µg/kg | **Sampling time** |
| | **Infusion:** 2 µg/kg/h | **Infusion:** 3 µg/kg/h | **Infusion:** 4 µg/kg/h | **Infusion:** 6 µg/kg/h | **Infusion:** 2 µg/kg/h | **Infusion:** 3 µg/kg/h | **Infusion:** 4 µg/kg/h | **Infusion:** 6 µg/kg/h | |
| **0.00** | 0.000 | 0.000 | 0.000 | 0.000 | 0.000 | 0.000 | 0.000 | 0.000 | 0 |
| **0.083** | 2.250 | 2.430 | 2.610 | 2.970 | 3.195 | 3.375 | 3.554 | 3.914 | 5 minutes |
| **0.167** | 1.148 | 1.372 | 1.596 | 2.045 | 1.498 | 1.722 | 1.946 | 2.395 | 10 minutes |
| **0.50** | 0.973 | 1.274 | 1.574 | 2.175 | 1.159 | 1.460 | 1.760 | 2.361 | 30 minutes |
| **1.00** | 0.985 | 1.359 | 1.734 | 2.484 | 1.102 | 1.477 | 1.852 | 2.601 | 1 hour |
| **2.00** | 1.007 | 1.460 | 1.913 | 2.819 | 1.057 | 1.510 | 1.963 | 2.869 | 2 hours |
| **3.00** | 1.027 | 1.515 | 2.004 | 2.981 | 1.052 | 1.540 | 2.029 | 3.006 | 3 hours |
| **4.00** | 1.046 | 1.554 | 2.062 | 3.077 | 1.061 | 1.569 | 2.077 | 3.092 | 4 hours |
| **6.00** | 1.081 | 1.612 | 2.142 | 3.204 | 1.090 | 1.621 | 2.152 | 3.214 | 6 hours |
| **8.00** | 1.111 | 1.659 | 2.207 | 3.303 | 1.119 | 1.667 | 2.215 | 3.310 | 8 hours |
| **11.92** | 1.161 | 1.736 | 2.310 | 3.459 | 1.167 | 1.742 | 2.316 | 3.465 | 11.92 hours |
| **12.03** | 0.946 | 1.413 | 1.880 | 2.814 | 0.952 | 1.419 | 1.886 | 2.819 | 2 minutes post-infusion |
| **12.08** | 0.803 | 1.199 | 1.594 | 2.386 | 0.809 | 1.205 | 1.600 | 2.392 | 5 minutes post-infusion |
| **12.17** | 0.716 | 1.068 | 1.419 | 2.123 | 0.722 | 1.073 | 1.425 | 2.129 | 10 minutes post-infusion |
| **12.25** | 0.670 | 0.998 | 1.327 | 1.985 | 0.675 | 1.004 | 1.333 | 1.991 | 15 minutes post-infusion |
| **12.50** | 0.567 | 0.844 | 1.122 | 1.677 | 0.573 | 0.850 | 1.128 | 1.683 | 30 minutes post-infusion |
| **13.00** | 0.424 | 0.630 | 0.836 | 1.248 | 0.429 | 0.635 | 0.842 | 1.254 | 1 hour post-infusion |
| **14.00** | 0.277 | 0.411 | 0.544 | 0.811 | 0.282 | 0.416 | 0.549 | 0.816 | 2 hours post-infusion |
| **16.00** | 0.186 | 0.275 | 0.363 | 0.540 | 0.191 | 0.279 | 0.368 | 0.545 | 4 hours post-infusion |
| **20.00** | 0.137 | 0.202 | 0.267 | 0.397 | 0.141 | 0.206 | 0.270 | 0.400 | 8 hours post-infusion |
| **24.00** | 0.107 | 0.157 | 0.208 | 0.309 | 0.110 | 0.160 | 0.211 | 0.312 | 12 hours post-infusion |
| **36.00** | 0.051 | 0.075 | 0.099 | 0.147 | 0.052 | 0.076 | 0.100 | 0.148 | 24 hours post-infusion |
| **48.00** | 0.024 | 0.036 | 0.047 | 0.070 | 0.025 | 0.036 | 0.048 | 0.070 | 36 hours post-infusion |
| **Peak bolus (ng/mL)** | 8.402 | 8.436 | 8.471 | 8.541 | 12.568 | 12.602 | 12.637 | 12.707 | |
| **C_{Max} infusion (ng/mL)** | 8.402 | 8.436 | 8.471 | 8.541 | 12.568 | 12.602 | 12.637 | 12.707 | |
| **T_{Max} Infusion (hours)** | 11.92 | 11.92 | 11.92 | 11.92 | 11.92 | 11.92 | 11.917 | 11.917 | |
| **Plateau 4-12 hours (ng/mL)** | 1.100 | 1.640 | 2.180 | 3.261 | 1.109 | 1.650 | 2.190 | 3.271 | |
| **Valley (ng/mL)** | 0.973 | 1.274 | 1.574 | 2.045 | 1.052 | 1.460 | 1.760 | 2.361 | |
| **T Valley (hours)** | 0.5 | 0.5 | 0.5 | 0.167 | 3 | 0.5 | 0.5 | 0.5 | |
| **% Dev. Plateau *versus* End of infusion** | 5.29 | 5.51 | 5.63 | 5.74 | 4.95 | 5.29 | 5.46 | 5.63 | |
| **% Dev. Valley *versus* Plateau** | 11.51 | 22.34 | 27.81 | 37.29 | 5.18 | 11.51 | 19.62 | 27.81 | |
| **ratio Peak bolus *versus* Plateau** | 7.64 | 5.14 | 3.89 | 2.62 | 11.33 | 7.64 | 5.77 | 3.89 | |
| **AUC 0-12 hours** | 12.89 | 18.87 | 24.78 | 36.68 | 13.35 | 19.34 | 25.32 | 37.28 | |
| **AUC 0-24 hours** | 15.51 | 22.74 | 29.91 | 44.32 | 16.02 | 23.26 | 30.50 | 44.97 | |

### Example 5 : metabolization of AOL - rats vs. sheep vs. human

As previously mentioned, AOL is shortly metabolized upon administration into its principal metabolite, desmethylanethole trithione (AOX). However, we have observed that metabolization of AOL is different in humans and in animals, which made it complicated if not impossible to readily translate data obtained in rats or sheep to human.

In more details, we demonstrated in the course of the pharmacokinetic study of Example 3 that AOL was rapidly metabolized into AOX, which quickly appeared in the plasma of human subjects after the start of the administration (**Fig. 9A**).

This starkly contrasted with the metabolization profile that we had observed in sheep (Example 2), where AOX remained below the limit of quantification (0.5 ng/mL) after bolus/infusion, despite similar or even higher doses (**Figs. 9B** **&** **9C**).

We also observed this inter-species difference in rat, with a metabolization profile that differed from that of both sheep and humans: after a bolus of 175 µg/kg and an infusion of 1.4 mg/kg/hr for 2 hours, AOX was detectable in the rat's plasma at 2 hours post-initiation, unlike in sheep; but the plasma levels of AOL (195 ng/mL) remained higher than that of AOX (94 ng/mL), unlike in humans.

### Example 6: two-step infusion

As explained in Example 3, the pharmacokinetics profile of AOL is characterized by a peak, followed by a valley (or trough), and finally a plateau corresponding to the achievement of steady-state AOL concentration.

Given the nature of ischemia-reperfusion injuries, in which an excessive production of mitochondrial ROS species occurs directly at reperfusion with significant tissue damage and necrosis, there is a need to reach the minimal target threshold of 1 ng of AOL per mL of plasma as fast as possible.

In particular, the specific dosage regimen administered in humans must be such that the trough observed after the initial peak does not drop below the target concentration threshold. The specific regimen of both the bolus and infusion are thus crucial.

In that respect, the management of the immediate post-initiation period has been evaluated using a 2-step infusion approach, *i.e.,* starting with a higher dose (6 µg/kg/h) in the first 3 hours of infusion, and then stepping down to a lower dose (3 µg/kg/h) for the remaining time (in this example, 9 hours, for a total infusion period of 12 hours).

The same 2-step infusion regimen was tested with 2 different bolus doses.

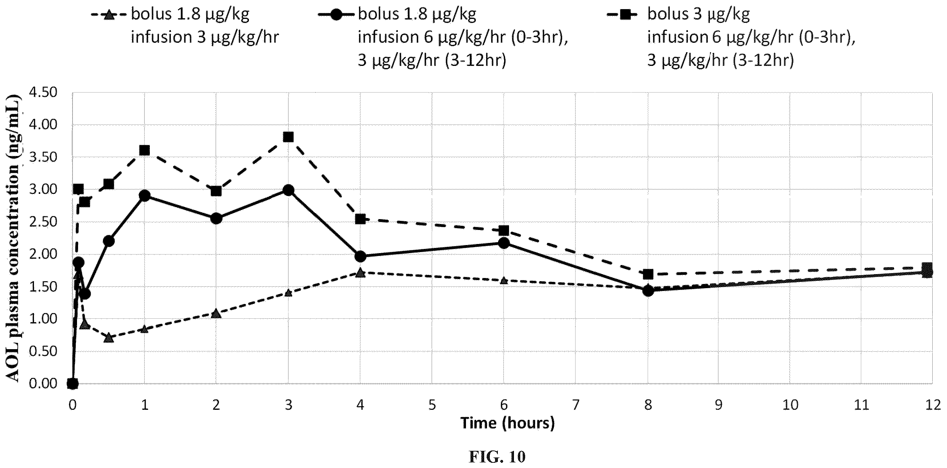

We could observe that this 2-step infusion paradigm abolished the trough observed immediately after administration of the bolus, at both the lower and higher bolus doses (**Fig. 10**).

In conclusion, we have identified three minimal dosage regimens that, in human, allowed to reach the target concentration threshold of 1 ng of AOL per mL of plasma rapidly through a bolus injection, without dropping below this concentration threshold during the first hours of the infusion:
i) a minimal bolus of 2 µg/kg immediately followed by a minimal infusion of 3 µg/kg/h (*cf.* **Table 3**); or
ii) a minimal bolus of 3 µg/kg immediately followed by a minimal infusion of 2 µg/kg/h (*cf.* **Table 3**); or
iii) a minimal bolus of 1.8 µg/kg immediately followed by a minimal infusion of 6 µg/kg/h for 3 hours then 3 µg/kg/h for the remaining time of infusion (*cf.* **Fig. 10**).

### Example 7: minimal duration of infusion

The minimal period of time of the infusion of AOL is critical in order to maintain the target concentration threshold. This duration was determined through modelling and simulation work.

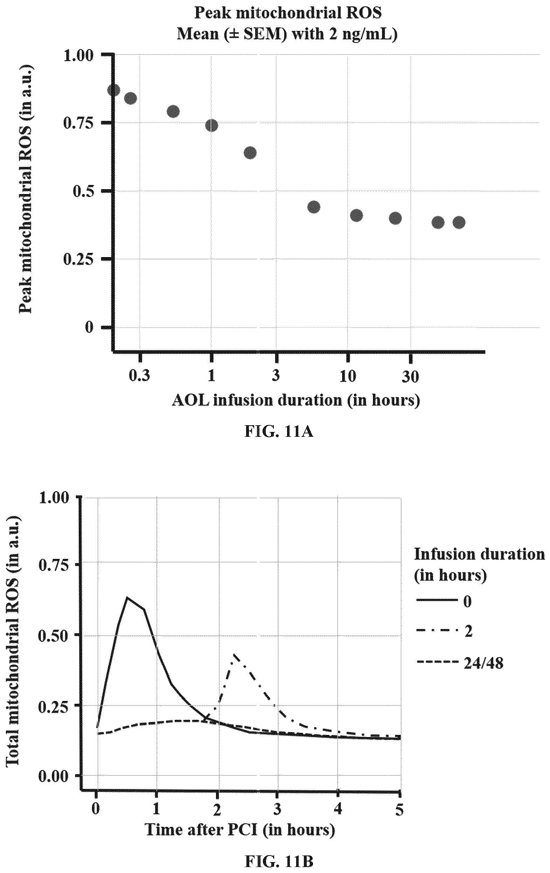

We have determined that a minimum mean duration of at least 6 hours was adequate to obtain an optimal mitochondrial ROS production inhibition (**Fig. 11A**). Below this duration, the inhibition of pathological mitochondrial ROS production was not complete, and resulted in an unwanted burst of ROS production immediately following the end of the infusion (**Fig. 11B**).

### Example 8: maximal target threshold of AOL

The maximal target threshold of AOL per mL of plasma in human, if not critical, can bear a particular importance: in pharmacology, it is indeed highly desired not to unnecessarily overload the patient's circulatory system for at least two reasons:
- to avoid any unwanted secondary effect, whether due to AOL itself or to any excipient in AOL's formulation; and/or
- to avoid any hypervolemia due to a too high volume of infused drug.

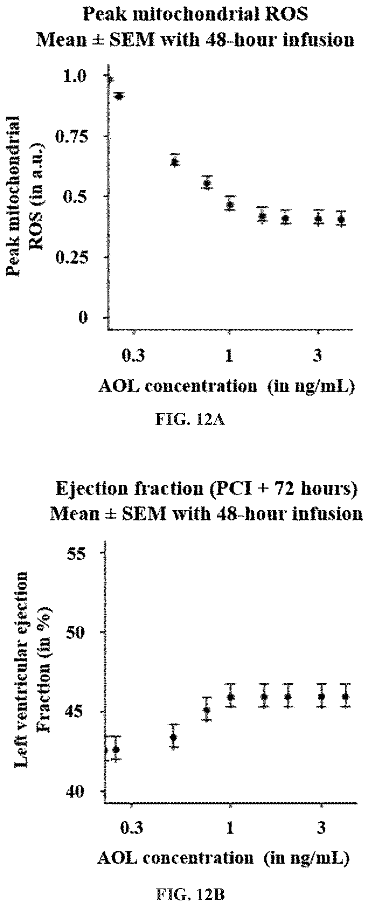

We have determined that a maximal target threshold at steady state was around 2-3 ng of AOL per mL of plasma. Above this concentration, no additional beneficial effect could be observed (**Figs. 12A & 12B**).

## Claims

1. Anethole trithione (AOL), for use in a method of preventing, treating, or lessening the severity or progression of, an ischemia-reperfusion injury in a human subject in need thereof, wherein:
a) a bolus of AOL is to be administered to the subject, said bolus comprising from 1.8 µg to 3 µg of AOL per kilogram (kg) of the subject's weight, and
b) an intravenous (IV) infusion of AOL is to be administered to the subject immediately after the bolus of AOL, said IV infusion comprising from 2 µg to 6 µg of AOL per kg of the subject's weight per hour, to be administered continuously for a period of time of 6 hours or more,
wherein the concentration of AOL in the subject's plasma at steady-state during infusion is above 1 ng of AOL per milliliter (mL) of subject's plasma, preferably the concentration of AOL in the subject's plasma at steady-state is assessed by liquid chromatography-mass spectrometry (LC/MS).

2. AOL for use according to claim **1,** wherein:
(i) the bolus of AOL comprises at least 2 µg of AOL per kg of the subject's weight, and the infusion comprises at least 3 µg of AOL per kg of the subject's weight per hour to be administered continuously for a period of time of 6 hours or more; or
(ii) the bolus of AOL comprises at least 3 µg of AOL per kg of the subject's weight, and the infusion comprises at least 2 µg of AOL per kg of the subject's weight per hour to be administered continuously for a period of time of 6 hours or more; or
(iii) the bolus of AOL comprises at least 1.8 µg of AOL per kg of the subject's weight, and the infusion comprises 6 µg of AOL per kg of the subject's weight per hour to be administered continuously for a first period of time of 3 hours, then at least 2 µg, preferably at least 3 µg of AOL per kg of the subject's weight per hour to be administered continuously for a second period of time of 3 hours or more.

3. AOL for use according to claim **1** or **2,** wherein the concentration of AOL in the subject's plasma at steady-state during infusion ranges from 1 ng to 3 ng of AOL per mL of subject's plasma, preferably from 1 ng to 2.25 ng of AOL per mL of subject's plasma, preferably the concentration of AOL in the subject's plasma at steady-state is assessed by liquid chromatography-mass spectrometry (LC/MS).

4. AOL for use according to any one of claims **1** to **3,** wherein the bolus of AOL is to be administered prior to or at the time of reperfusion.

5. AOL for use according to any one of claims **1** to **4,** wherein the bolus of AOL is to be administered intravenously or intra-arterially, preferably by intracoronary injection.

6. AOL for use according to any one of claims **1** to **5,** wherein the infusion of AOL is to be administered continuously for 6 hours to 24 hours.

7. AOL for use according to any one of claims **1** to **6,** wherein the ischemia-reperfusion injury occurs after ischemia and reperfusion of a tissue selected from the group comprising cardiac muscle tissue, skin tissue, skeletal muscle tissue, smooth muscle tissue, cartilage tissue, tendon tissue, brain tissue, spinal cord tissue, retinal tissue, corneal tissue, lung tissue, liver tissue, kidney tissue, pancreatic tissue, ovary tissue, testis tissue, intestinal tissue, stomach tissue, bladder tissue, or distal limb tissue.

8. AOL for use according to any one of claims **1** to **7,** wherein the ischemia-reperfusion injury occurs after ischemia and reperfusion of the heart and wherein the ischemia-reperfusion injury is an acute coronary syndrome.

9. AOL for use according to claim **8,** wherein the acute coronary syndrome is selected from the group comprising or consisting of ST elevation myocardial infarction (STEMI), non-ST elevation myocardial infarction (NSTEMI) and unstable angina.

10. AOL for use according to claim **8** or **9,** wherein the acute coronary syndrome is STEMI.

11. AOL for use according to any one of claims **8** to **10,** wherein preventing, treating, or lessening the severity or progression of the acute coronary syndrome comprises one or more of reducing reperfusion-induced ST-segment elevation, reducing troponin Ic release, reducing creatine phosphokinase (CPK) release, reducing infarct size, and increasing left ventricular ejection fraction.

12. AOL for use according to any one of claims **1** to **11,** wherein the subject is undergoing surgery at the time of ischemia.

13. AOL for use according to claim **12,** wherein surgery is selected from the group comprising artery clamping and angioplasty.

14. AOL for use according to any one of claims **1** to **11,** wherein the subject is undergoing tissue or organ transplantation at the time of ischemia.

15. AOL for use according to claim **14,** wherein tissue or organ transplantation is coronary artery bypass graft surgery.

## Patentansprüche

1. Anetholtrithion (AOL) zur Verwendung in einem Verfahren zur Vorbeugung, Behandlung oder Minderung des Schweregrads oder des Fortschreitens einer Ischämie-Reperfusionsschädigung bei einem menschlichen Probanden, der dies benötigt, wobei:
a) dem Probanden ein Bolus von AOL verabreicht werden soll, wobei der Bolus 1,8 µg bis 3 µg AOL pro Kilogramm (kg) des Körpergewichts des Probanden umfasst, und
b) dem Probanden unmittelbar nach dem Bolus von AOL eine intravenöse (IV) Infusion von AOL verabreicht werden soll, wobei die IV-Infusion 2 µg bis 6 µg AOL pro kg des Körpergewichts des Probanden pro Stunde umfasst und kontinuierlich über einen Zeitraum von 6 Stunden oder mehr verabreicht werden soll,
wobei die Konzentration von AOL in dem Steady-State-Plasma des Probanden während der Infusion über 1 ng AOL pro Milliliter (ml) des Plasmas des Probanden liegt, wobei vorzugsweise die Konzentration von AOL in dem Steady-State-Plasma des Probanden durch Flüssigkeitschromatographie-Massenspektrometrie (LC/MS) beurteilt wird.

2. AOL zur Verwendung nach Anspruch 1, wobei:
(i) der Bolus von AOL mindestens 2 µg AOL pro kg des Körpergewichts des Probanden umfasst und die Infusion mindestens 3 µg AOL pro kg des Körpergewichts des Probanden pro Stunde umfasst und kontinuierlich über einen Zeitraum von 6 Stunden oder mehr verabreicht werden soll; oder
(ii) der Bolus von AOL mindestens 3 µg AOL pro kg des Körpergewichts des Probanden umfasst und die Infusion mindestens 2 µg AOL pro kg des Körpergewichts des Probanden pro Stunde umfasst und kontinuierlich über einen Zeitraum von 6 Stunden oder mehr verabreicht werden soll; oder
(iii) der Bolus von AOL mindestens 1,8 µg AOL pro kg des Körpergewichts des Probanden umfasst und die Infusion 6 µg AOL pro kg des Körpergewichts des Probanden pro Stunde umfasst und kontinuierlich über einen ersten Zeitraum von 3 Stunden verabreicht werden soll, dann mindestens 2 µg, vorzugsweise mindestens 3 µg AOL pro kg des Körpergewichts des Probanden pro Stunde umfasst und kontinuierlich über einen zweiten Zeitraum von 3 Stunden oder mehr verabreicht werden soll.

3. AOL zur Verwendung nach Anspruch **1** oder **2,** wobei die Konzentration von AOL in dem Steady-State-Plasma des Probanden während der Infusion zwischen 1 ng und 3 ng AOL pro mL Plasma des Probanden und vorzugsweise zwischen 1 ng und 2,25 ng AOL pro mL Plasma des Probanden liegt, wobei vorzugsweise die Konzentration von AOL in dem Steady-State-Plasma des Probanden durch Flüssigkeitschromatographie-Massenspektrometrie (LC/MS) beurteilt wird.

4. AOL zur Verwendung nach einem der Ansprüche **1** bis **3,** wobei der Bolus von AOL vor oder zu dem Zeitpunkt der Reperfusion verabreicht werden soll.

5. AOL zur Verwendung nach einem der Ansprüche **1** bis **4,** wobei der Bolus von AOL intravenös oder intra-arteriell verabreicht werden soll, vorzugsweise durch intrakoronare Injektion.

6. AOL zur Verwendung nach einem der Ansprüche **1** bis **5,** wobei die Infusion von AOL kontinuierlich 6 Stunden bis 24 Stunden lang verabreicht werden soll.

7. AOL zur Verwendung nach einem der Ansprüche **1** bis **6,** wobei die Ischämie-Reperfusionsschädigung nach Ischämie und Reperfusion eines Gewebes auftritt, das aus der Gruppe ausgewählt wird, die Herzmuskelgewebe, Hautgewebe, Skelettmuskelgewebe, glattes Muskelgewebe, Knorpelgewebe, Sehnengewebe, Gehirngewebe, Rückenmarkgewebe, Netzhautgewebe, Hornhautgewebe, Lungengewebe, Lebergewebe, Nierengewebe, Pankreasgewebe, Eierstockgewebe, Hodengewebe, Darmgewebe, Magengewebe, Blasengewebe oder Gewebe der distalen Gliedmaßen umfasst.

8. AOL zur Verwendung nach einem der Ansprüche **1** bis **7,** wobei die Ischämie-Reperfusionsschädigung nach Ischämie und Reperfusion des Herzens auftritt und wobei die Ischämie-Reperfusionsschädigung ein akutes Koronarsyndrom ist.

9. AOL zur Verwendung nach Anspruch **8,** wobei das akute Koronarsyndrom aus der Gruppe ausgewählt wird, die ST-Hebungs-Myokardinfarkt (STEMI), Nicht-ST-Hebungs-Myokardinfarkt (NSTEMI) und instabiler Angina umfasst oder daraus besteht.

10. AOL zur Verwendung nach Anspruch **8** oder **9,** wobei das akute Koronarsyndrom STEMI ist.

11. AOL zur Verwendung nach einem der Ansprüche **8** bis **10,** wobei die Vorbeugung, Behandlung oder Minderung des Schweregrads oder des Fortschreitens des akuten Koronarsyndroms eines oder mehrere von einer Reduzierung der durch Reperfusion induzierten ST-Segmenthebung, einer Reduzierung der Troponin-Ic-Freisetzung, einer Reduzierung der Kreatinphosphokinase (CPK)-Freisetzung, einer Reduzierrung der Infarktgröße und einer Erhöhung der linksventrikulären Ejektionsfraktion umfasst.

12. AOL zur Verwendung nach einem der Ansprüche **1** bis **11,** wobei sich der Proband zu dem Zeitpunkt der Ischämie einer Operation unterzieht.

13. AOL zur Verwendung nach Anspruch **12,** wobei die Operation aus der Gruppe ausgewählt wird, die Arterienabklemmung und Angioplastie umfasst.

14. AOL zur Verwendung nach einem der Ansprüche **1** bis **11,** wobei sich der Proband zu dem Zeitpunkt der Ischämie einer Gewebe- oder Organtransplantation unterzieht.

15. AOL zur Verwendung nach Anspruch **14,** wobei die Gewebe- oder Organtransplantation die Koronararterien-Bypass-Operation ist.

## Revendications

1. Anéthole trithione (AOL), pour son utilisation dans une méthode de prévention, de traitement ou de diminution de la sévérité ou de la progression d'une lésion d'ischémie-reperfusion chez un sujet humain qui le nécessite, où :
a) un bolus d'AOL est à administrer au sujet, ledit bolus comprenant de 1,8 µg à 3 µg d'AOL par kilogramme (kg) du poids du sujet, et
b) une infusion intraveineuse (IV) d'AOL est à administrer au sujet immédiatement après le bolus d'AOL, ladite infusion (IV) comprenant de 2 µg à 6 µg d'AOL par kg du poids du sujet par heure, à administrer de façon continue sur une période de 6 heures ou plus,
**caractérisé en ce que** la concentration d'AOL dans le plasma du sujet à l'état d'équilibre pendant l'infusion est supérieure à 1 ng d'AOL par millilitre (mL) de plasma de sujet, de préférence la concentration d'AOL dans le plasma du sujet à l'état d'équilibre est déterminée par chromatographie liquide-spectrométrie de masse (LC/MS).

2. AOL pour son utilisation selon la revendication **1, caractérisé en ce que** :
(i) le bolus d'AOL comprend au moins 2 µg d'AOL par kg du poids du sujet, et l'infusion comprend au moins 3 µg d'AOL par kg du poids du sujet par heure à administrer de façon continue sur une période de 6 heures ou plus ; ou
(ii) le bolus d'AOL comprend au moins 3 µg d'AOL par kg du poids du sujet, et l'infusion comprend au moins 2 µg d'AOL par kg du poids du sujet par heure à administrer de façon continue sur une période de 6 heures ou plus ; ou
(iii) le bolus d'AOL comprend au moins 1,8 µg d'AOL par kg du poids du sujet, et l'infusion comprend au moins 6 µg d'AOL par kg du poids du sujet par heure à administrer de façon continue sur une première période de 3 heures, puis au moins 2 µg, de préférence au moins 3 µg d'AOL par kg du poids du sujet par heure à administrer de façon continue sur une seconde période de 3 heures ou plus.

3. AOL pour son utilisation selon la revendication **1** ou **2, caractérisé en ce que** la concentration d'AOL dans le plasma du sujet à l'état d'équilibre pendant l'infusion varie de 1 ng à 3 ng d'AOL par mL de plasma du sujet, de préférence de 1 ng à 2,25 ng d'AOL par mL de plasma du sujet, de préférence la concentration d'AOL dans le plasma du sujet à l'état d'équilibre est déterminée par chromatographie liquide-spectrométrie de masse (LC/MS).

4. AOL pour son utilisation selon l'une quelconque des revendications **1** à **3, caractérisé en ce que** le bolus d'AOL est à administrer avant ou au moment de la reperfusion.

5. AOL pour son utilisation selon l'une quelconque des revendications **1** à **4, caractérisé en ce que** le bolus d'AOL est à administrer par voie intraveineuse ou intra-artérielle, de préférence par injection intracoronaire.

6. AOL pour son utilisation selon l'une quelconque des revendications **1** à **5, caractérisé en ce que** l'infusion d'AOL est à administrer de façon continue pendant 6 heures à 24 heures.

7. AOL pour son utilisation selon l'une quelconque des revendications **1** à **6, caractérisé en ce que** la lésion d'ischémie-reperfusion se produit après l'ischémie et la reperfusion d'un tissu sélectionné dans le groupe parmi le tissu du muscle cardiaque, le tissu de la peau, le tissu du muscle squelettique, le tissu du muscle lisse, le tissu du cartilage, le tissu du tendon, le tissu du cerveau, le tissu de la moelle épinière, le tissu rétinien, le tissu cornéen, le tissu du poumon, le tissu du foie, le tissu du rein, le tissu pancréatique, le tissu ovarien, le tissu des testicules, le tissu intestinal, le tissu de l'estomac, le tissu de la vessie et le tissu du membre distal.

8. AOL pour son utilisation selon l'une quelconque des revendications **1** à **7, caractérisé en ce que** la lésion d'ischémie-reperfusion se produit après l'ischémie et la reperfusion du cœur et **caractérisé en ce que** la lésion d'ischémie-reperfusion est un syndrome coronarien aigu.

9. AOL pour son utilisation selon la revendication **8, caractérisé en ce que** le syndrome coronarien aigu est sélectionné dans le groupe comprenant ou consistant en l'infarctus du myocarde avec surélévation du segment ST (STEMI), l'infarctus du myocarde sans surélévation du segment ST (NSTEMI) et l'angine instable.

10. AOL pour son utilisation selon la revendication **8** ou la revendication **9, caractérisé en ce que** le syndrome coronarien aigu est le STEMI.

11. AOL pour son utilisation selon l'une quelconque des revendications **8** à **10, caractérisé en ce que** la prévention, le traitement ou la diminution de la sévérité ou de la progression du syndrome coronarien aigu comprend un ou plus de la réduction de la surélévation du segment ST induite par la reperfusion, la réduction de la libération de troponine Ic, la réduction de la libération de créatine phosphokinase (CPK), la réduction de la taille de l'infarctus, et l'augmentation de la fraction d'éjection ventriculaire gauche.

12. AOL pour son utilisation selon l'une quelconque des revendications **1** à **11, caractérisé en ce que** le sujet est en train de subir une intervention chirurgicale au moment de l'ischémie.

13. AOL pour son utilisation selon la revendication **12, caractérisé en ce que** l'intervention chirurgicale est sélectionnée dans le groupe comprenant le clampage de l'artère et l'angioplastie.

14. AOL pour son utilisation selon l'une quelconque des revendications **1** à **11, caractérisé en ce que** le sujet est en train de subir une transplantation de tissu ou d'organe au moment de l'ischémie.

15. AOL pour son utilisation selon la revendication **14, caractérisé en ce que** la transplantation de tissu ou d'organe est une intervention chirurgicale de pontage aortocoronarien.
